# EUROPÄISCHE PATENTSCHRIFT

(11) **EP 2 276 845 B1**
(45) Veröffentlichungstag und Bekanntmachung des Hinweises auf die Patenterteilung: **22.01.2014**
(21) Anmeldenummer: 09749820.8
(22) Anmeldetag: 19.05.2009
(51) Int. Cl.: C12P 13/04, C12P 13/08, C07K 14/35, C12N 15/31, C12N 15/77, C12R 1/15

(54) **VERFAHREN ZUR HERSTELLUNG VON L-AMINOSÄUREN**
METHOD FOR MANUFACTURING L-AMINO ACIDS
PROCÉDÉ POUR PRODUIRE DES ACIDES AMINÉS L

(30) Priorität: 20.05.2008 DE 102008001874
(43) Veröffentlichungstag der Anmeldung: 26.01.2011
(73) Patentinhaber: Evonik Degussa GmbH, 45128 Essen (DE)
(72) Erfinder: JESSBERGER, Nadja, 91074 Herzogenaurach (DE); BURKOVSKI, Andreas, 91052 Erlangen (DE); BATHE, Brigitte, 33154 Salzkotten (DE); RETH, Alexander, 33615 Bielefeld (DE)
(86) Internationale Anmeldenummer: PCT/EP2009/056046
(87) Internationale Veröffentlichungsnummer: WO 2009/141330

(56) Entgegenhaltungen:
- EP-A- 1 460 128
- BECKERS GABRIELE ET AL: "Regulation of AmtR-controlled gene expression in Corynebacterium glutamicum: mechanism and characterization of the AmtR regulon." MOLECULAR MICROBIOLOGY OCT 2005, Bd. 58, Nr. 2, Oktober 2005 (2005-10), Seiten 580-595, XP002539889 ISSN: 0950-382X

## Beschreibung

Gegenstand der Erfindung ist ein Verfahren zur Herstellung von L-Aminosäuren unter Verwendung von coryneformen Bakterien, in denen der AmtR-Regulator abgeschwächt worden ist.

### Stand der Technik

L-Aminosäuren findet in der Humanmedizin, in der pharmazeutischen Industrie, in der Lebensmittelindustrie und ganz besonders in der Tierernährung Anwendung.

Es ist bekannt, dass L-Aminosäuren, wie beispielsweise das L-Lysin, durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die L-Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen der Gattung Corynebacterium, insbesondere Corynebacterium glutamicum, eingesetzt, indem man einzelne Aminosäure-Biosynthesegene modifiziert und die Auswirkung auf die Aminosäure-Produktion untersucht.

Zusammenfassende Darstellungen zur Biologie, Genetik und Biotechnologie von Corynebacterium glutamicum sind im "Handbook of Corynebacterium glutamicum" (Eds.: L. Eggeling und M. Bott, CRC Press, Taylor & Francis, 2005), in der Spezialausgabe des Journal of Biotechnolgy (Chief Editor: A. Pühler) mit dem Titel "A New Era in Corynebacterium glutamicum Biotechnology" (Journal of Biotechnolgy 104/1-3, (2003)) und im Buch von T. Scheper (Managing Editor) "Microbial Production of L-Amino Acids" (Advances in Biochemical Engineering/Biotechnology 79, Springer Verlag, Berlin, Deutschland, 2003) zu finden.

Die Nukleotidsequenz des Genoms von Corynebacterium glutamicum ATCC13032 ist bei Ikeda und Nakagawa (Applied Microbiology and Biotechnology 62, 99-109 (2003)), in der EP 1 108 790 und bei Kalinowski et al. (Journal of Biotechnolgy 104(1-3), (2003)) beschrieben. Die Nukleotidsequenz des Genoms von Corynebacterium glutamicum R ist bei Yukawa et al. (Microbiology 153(4):1042-1058 (2007)) beschrieben.

Die Nukleotidsequenz des Genoms von Corynebacterium efficiens ist bei Nishio et al (Genome Research. 13 (7), 1572-1579 (2003)) beschrieben.

Die Nukleotidsequenzen des Genoms von Corynebacterium glutamicum und Corynebacterium efficiens sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Die Entschlüsselung des Corynebacterium glutamicum Genoms ermöglichte unter Anderem weitreichende Untersuchungen zum Stoffwechsel und regulatorischen Netzwerk dieses Bakteriums (Silberbach und Burkovski, Journal of Biotechnology 126(1): 101-110 (2006)).

Eine wesentliche Vorraussetzung für die Synthese von Aminosäuren sowie generell für die Vermehrung der Zellen ist eine adäquate Versorgung mit Stickstoff. C. glutamicum ist in der Lage, verschiedene Stickstoffquellen, darunter Ammonium, L-Glutaminsäure, Glutamin und Harnstoff, zu verwerten. In Abhängigkeit von Konzentration und Art der verfügbaren Stickstoffquelle werden bestimmte Enzyme und Transportsysteme synthetisiert bzw. aktiviert. Aus energetischen Gründen ist eine strikte Regulation ("Stickstoff-Kontrolle") notwendig. Die Regulation der Genexpression und die globale Signaltransduktion im Stickstoff-Metabolismus von C. glutamicum wurde von verschiedenen Autoren detailliert untersucht.

Die Expression Stickstoff-regulierter Gene wird in C. glutamicum durch den globalen Repressor AmtR reguliert. AmtR reprimiert bei guter Stickstoff-Versorgung die Expression der Gene des amt-soxA-ocd-Operons, des gltBD-Operons, des amtB-glnK-glnD-Operons sowie der Gene glnA und crnT (Jacoby et al., Molecular Microbiology 37: 964-977 (2000); Beckers et al., Microbiology, 147: 2961-2170 (2001); Nolden et al., FEMS Microbiological Letters 201: 91-98 (2001)). Bei weiteren Untersuchungen (Beckers et al., Journal of Bacteriology 186(22): 7645-52 (2004); Beckers et al., Molecular Microbiology 58(2): 580-595 (2005)) konnte unter Anderem auch für die Gene des gluABCD-Operons, des NCg11915-1918-Operons, des urtABCDE-Operons, des ureABCEFGD-Operons sowie das codA-Gen und das NCg11099-Gen eine AmtR-abhängige Regulation gezeigt werden.

In der EP 1 460 128 wird über die Auswirkung der Deletion des AmtR-Gens in einem ΔargR-Stamm auf die Produktion verschiedener Aminosäuren berichtet.

Die Regulierung der AmtR abhängigen Genexpression in Corynebakterium glutamicum wird in Beckers, Gabriele et al.: "Regulation of AmtR-controlled gene expression in Corynebacterium glutamicum: mechanism and characterization oft he AmtR regulon", Molecular Microbiology (2005) 58(2), 580-595 offenbart.

### Aufgabe der Erfindung

Die Erfinder haben sich zur Aufgabe gestellt neue Maßnahmen zur verbesserten Herstellung von L-Aminosäuren, bereitzustellen.

### Beschreibung der Erfindung

Gegenstand der Erfindung ist ein rekombinantes, L-Aminosäure ausscheidendes, coryneformes Bakterium bei dem das amtR-Gen, das für einen AmtR-Regulator kodiert, dessen Aminosäuresequenz zu mindestens 90%, bevorzugt zu mindestens 95%, besonders bevorzugt zu mindestens 98% oder zu mindestens 99% und ganz besonders bevorzugt identisch ist mit der Aminosäuresequenz von SEQ ID NO:2 und im Wesentlichen eine Länge von 222 Aminosäuren umfasst, abgeschwächt worden ist, durch Austausch des Glycin an Position 3 der Aminosäuresequenz gegen L-Glutaminsäure

Für die Herstellung der erfindungsgemäßen Bakterien werden coryneforme Bakterien eingesetzt. Unter den coryneformen Bakterien wird die Gattung Corynebacterium bevorzugt. Innerhalb der Gattung Corynebacterium werden Stämme bevorzugt, die auf folgenden Arten beruhen:
Corynebacterium efficiens, wie zum Beispiel der Typstamm DSM44549,
Corynebacterium glutamicum, wie zum Beispiel der Typstamm ATCC13032 oder der Stamm R, und
Corynebacterium ammoniagenes, wie zum Beispiel der Stamm ATCC6871,
wobei die Art Corynebacterium glutamicum ganz besonders bevorzugt wird.

Einige Vertreter der Art Corynebacterium glutamicum sind im Stand der Technik auch unter anderen Bezeichnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869, und
Brevibacterium divaricatum ATCC14020.

Der Begriff "Micrococcus glutamicus" für Corynebacterium glutamicum war ebenfalls gebräuchlich.

Einige Vertreter der Art Corynebacterium efficiens wurden im Stand der Technik auch als Corynebacterium thermoaminogenes bezeichnet, wie zum Beispiel der Stamm FERM BP-1539.

Die für die Maßnahmen der Abschwächung eingesetzten Stämme coryneformer Bakterien (Ausgangsstämme) besitzen bevorzugt bereits die Fähigkeit die gewünschte(n) L-Aminosäure(en) in der Zelle anzureichern oder in das sie umgebende Nährmedium auszuscheiden und dort zu akkumulieren. Hierfür wird im Folgenden auch der Ausdruck "produzieren" verwendet. Insbesondere besitzen die für die Maßnahmen der Abschwächung eingesetzten Stämme coryneformer Bakterien die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Verbindung in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist offensichtlich und bedarf keiner weiteren Erklärungen, dass man zu einem erfindungsgemäßen Bakterium auch dadurch gelangen kann, indem man in einem Wildstamm, wie zum Beispiel in dem Typstamm ATCC13032 oder in dem Stamm ATCC14067, zunächst das amtR-Gen mit Hilfe der Maßnahmen der Erfindung abschwächt und anschließend durch geeignete weitere genetische Massnahmen, das Bakterium veranlasst, die gewünschte(n) L-Aminosäure(en) zu produzieren.

Der Begriff L-Aminosäuren umfasst die proteinogenen Aminosäuren, sowie L-Ornithin und L-Homoserin. Unter proteinogenen L-Aminosäuren versteht man die L-Aminosäuren die in natürlichen Proteinen, das heißt in Proteinen von Mikroorganismen, Pflanzen, Tieren und Menschen, vorkommen. Zu den proteinogenen Aminosäuren zählen L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin, L-Prolin und gegebenenfalls L-Selenocystein und L-Pyrrolysin. Bevorzugt werden die L-Aminosäuren L-Lysin, L-Glutaminsäure, L-Glutamin, L-Arginin, L-Prolin und L-Ornithin. Besonders bevorzugt wird L-Lysin.

Werden im Folgenden Aminosäuren oder L-Aminosäuren erwähnt, umfasst der Begriff auch deren Salze wie beispielsweise das Lysin-Monohydrochlorid oder Lysin-Sulfat im Falle der Aminosäure L-Lysin.

Bekannte Vertreter L-Lysin produzierender bzw. ausscheidender Stämme coryneformer Bakterien sind beispielsweise:
Corynebacterium glutamicum DM58-1/pDM6 (= DSM4697) beschrieben in EP 0 358 940,
Corynebacterium glutamicum MH20-22B (= DSM16835) beschrieben in Menkel et al. (Applied and Environmental Microbiology 55(3), 684-688 (1989)),
Corynebacterium glutamicum AHP-3 (= Ferm BP-7382) beschrieben in EP 1 108 790,
Corynebacterium glutamicum NRRL B-11474 beschrieben in US 4,275,157,
Corynebacterium glutamicum DSM13994 beschrieben in US 6,783,967
Corynebacterium glutamicum DSM16834 beschrieben in WO 06/063660,
Corynebacterium glutamicum DSM17119 beschrieben in WO 06/100211,
Corynebacterium glutamicum DSM17223 beschrieben in WO 06/125714,
Corynebacterium glutamicum DSM16937 beschrieben in WO 06/077004, und
Corynebacterium thermoaminogenes AJ12521 (= FERM BP-3304) beschrieben in US 5,250,423.

Angaben zur taxonomischen Einordnung von Stämmen der Gruppe der coryneformen Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)), Fudou et al (International Journal of Systematic and Evolutionary Microbiology 52, 1127-1131 (2002)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden.

L-Lysin produzierende coryneforme Bakterien besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform (Wildtyp) eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese im Vergleich zum Wildtyp desensibilisierten Aspartatkinasen kodierenden Gene bzw. Allele werden auch als lysC^{FBR}-Allele bezeichnet. Im Stand der Technik sind zahlreiche lysC^{FBR}-Allele beschrieben, die für Aspartatkinasevarianten kodieren, welche Aminosäureaustausche im Vergleich zum Wildtypprotein besitzen. Die Kodierregion des Wildtyp lysC-Gens von Corynebacterium glutamicum ATCC13032 entsprechend der Zugangsnummer AX756575 der NCBI Datenbank ist in SEQ ID NO:7 und das von diesem Gen kodierte Polypeptid in SEQ ID NO:8 dargestellt. Die Aminosäuresequenz der Wildform der Aspartatkinase variiert geringfügig bei verschiedenen Wildtypstämmen von Corynebacterium glutamicum. So enthält die Aspartatkinase des Wildtypstammes Corynebacterium glutamicum ATCC14067 an Position 317 Alanin. Die Wildtyp-Aspartatkinase von Stamm ATCC 13032 enthält an dieser Position Serin wie in SEQ ID NO:8 dargestellt.

Die für die Maßnahmen der Erfindung eingesetzten L-Lysin produzierenden coryneformen Bakterien verfügen bevorzugt über ein lysC-Allel, das für eine Aspartatkinasevariante kodiert, welche die Aminosäuresequenz von SEQ ID NO:13 besitzt, wobei diese eine oder mehrere der Aminosäureaustausche ausgewählt aus der Gruppe:
LysC A279T (Austausch von L-Alanin an Position 279 des kodierten Aspartatkinaseproteins gemäß SEQ ID NO: 13 gegen L-Threonin; siehe US 5,688,671 und Zugangsnummern E06825, E06826, E08178 und I74588 bis I74597),
LysC A279V (Austausch von L-Alanin an Position 279 des kodierten Aspartatkinaseproteins gemäß SEQ ID NO: 13 gegen L-Valin; siehe JP 6-261766 und Zugangsnummer E08179),
LysC L297Q (Austausch von L-Leucin an Position 297 des kodierten Aspartatkinaseproteins gemäß SEQ ID NO: 13 gegen L-Glutamin; siehe DE 102006026328,
LysC S301F (Austausch von L-Serin an Position 301 des kodierten Aspartatkinaseproteins gemäß SEQ ID NO: 13 gegen L-Phenylalanin; siehe US 6,844,176 und Zugangsnummer E08180),
LysC S301Y (Austausch von L-Serin an Position 301 des kodierten Aspartatkinaseproteins gemäß SEQ ID NO: 13 gegen L-Tyrosin; siehe Kalinowski et al. (Molecular and General Genetics 224, 317-324 (1990)) und Zugangsnummer X57226),
LysC T308I (Austausch von L-Threonin an Position 308 des kodierten Aspartatkinaseproteins gemäß SEQ ID NO: 13 gegen L-Isoleucin; siehe JP 6-261766 und Zugangsnummer E08181)
LysC T311I (Austausch von L-Threonin an Position 311 des kodierten Aspartatkinaseproteins gemäß SEQ ID NO: 13 gegen L-Isoleucin; siehe WO 00/63388 und US 6,893,848),
LysC R320G (Austausch von L-Arginin an Position 320 des kodierten Aspartatkinaseproteins gemäß SEQ ID NO: 13 gegen Glycin; siehe Jetten et al. (Applied Microbiology and Biotechnology 43, 76-82 (995)) und Zugangsnummer L27125),
LysC G345D (Austausch von Glycin an Position 345 des kodierten Aspartatkinaseproteins gemäß SEQ ID NO: 13 gegen L-Asparaginsäure; siehe Jetten et al. (Applied Microbiology and Biotechnology 43, 76-82 (995)) und Zugangsnummer L16848),
LysC T380I (Austausch von L-Threonin an Position 380 des kodierten Aspartatkinaseproteins gemäß SEQ ID NO: 13 gegen L-Isoleucin; siehe WO 01/49854 und Zugangsnummer AX192358), und
LysC S381F (Austausch von L-Serin an Position 381 des kodierten Aspartatkinaseproteins gemäß SEQ ID NO: 13 gegen L-Phenylalanin; siehe EP 0435132)
umfasst, wobei ggf. an Postion 317 anstelle L-Serin L-Alanin enthalten ist.

Besonders bevorzugt werden das lysC^{FBR}-Allel lysC T311I (Austausch von Threonin an Position 311 des kodierten Aspartatkinaseproteins gemäß SEQ ID NO: 13 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 13 gegen Threonin), S381F (Austausch von Serin an Position 381 des kodierten Aspartatkinaseproteins gemäß SEQ ID NO: 13 gegen Phenylalanin), wobei gegebenenfalls das Serin an Position 317 gegen Alanin ausgetauscht ist (S317A).

Ganz besonders bevorzugt wird das lysC^{FBR}-Allel lysC T311I (Austausch von Threonin an Position 311 des kodierten Aspartatkinaseproteins gemäß SEQ ID NO: 13 gegen Isoleucin), wobei gegebenenfalls das Serin an Position 317 gegen Alanin ausgetauscht ist (S317A).

Der Stamm DSM 16833 (WO 06/063660) besitzt ein lysC^{FBR}-Allel, das für ein Aspartatkinaseprotein kodiert, welches den Aminosäureaustausch T311I enthält.

Der Stamm NRRL B-11474 (US 4,275,157) besitzt ein lysC^{FBR}-Allel, das für ein Aspartatkinaseprotein kodiert, welches den Aminosäureaustausch S381F enthält.

Es hat sich weiterhin als vorteilhaft für die Lysinproduktion herausgestellt, die lysC^{FBR}-Allele zu überexprimieren.

In einer weiteren Ausführungsform besitzen die für die Massnahmen der Erfindung eingesetzten L-Lysin produzierenden Bakterien der Gattung Corynebacterium, die bevorzugt außerdem ein Polynukleotid enthalten, das für eine Lysin-insensitive Aspartatkinasevariante kodiert, eines oder mehrere der Merkmale ausgewählt aus der Gruppe:
a) überexprimiertes Polynukleotid (dapA-Gen), das für eine Dihydrodipicolinat-Synthase (DapA, EC Nr. 4.2.1.52)kodiert,
b) überexprimiertes Polynukleotid (asd-Gen), das für eine Aspartatsemialdehyd-Dehydrogenase (Asd, EC Nr. 1.2.1.11) kodiert,
c) überexprimiertes Polynukleotid (ddh-Gen), das für eine meso-Diaminopimelate Dehydrogenase (Ddh, EC Nr. 1.4.1.16) kodiert,
d) überexprimiertes Polynukleotid, (lysA-Gen), das für eine Diaminopimelat-Decarboxylase (LysA, EC Nr. 4.1.1.20) kodiert,
e) überexprimiertes Polynukleotid (aat-Gen), das für eine Aspartat-Aminotransferase (Aat, EC Nr. 2.6.1.1) kodiert,
f) überexprimiertes Polynukleotid (lysE-Gen), das für eine Polypeptid mit L-Lysin-Export Aktivität (LysE, Lysin Efflux Permease) kodiert,
g) überexprimiertes Polynukleotid, das für eine Pyruvat-Carboxylase (Pyc, EC Nr. 6.4.1.1) kodiert, und
h) überexprimiertes Polynukleotid (dapB-Gen), das für eine Dihydrodipicolinat-Synthase (DapB, EC Nr. 1.3.1.26) kodiert.

Hierfür können die im Stand der Technik beschriebenen Gene von Bakterien verwendet werden. Bevorzugt werden die endogenen Gene bzw. Polynukleotide der Gattung Corynebacterium, besonders bevorzugt die der Art Corynebacterium glutamicum, Corynebacterium efficiens und Corynebacterium ammoniagenes und ganz besonders bevorzugt die der Art Corynebacterium glutamicum verwendet.

Unter endogenen Genen beziehungsweise Polynukleotiden versteht man die in der Population einer Art vorhandenen offenen Leserahmen (ORF), Gene oder Allele beziehungsweise deren Polynukleotide.

Das dapA-Gen von Corynebacterium glutamicum Stamm ATCC13032 ist beispielsweise in der EP 0 197 335 beschrieben. Zur Überexpression des dapA-Gens von Corynebacterium glutamicum können außerdem unter anderem die Mutationen MC20 und MA16 des dapA-Promotors, wie in der US 6,861,246 beschrieben, eingesetzt werden.

Das asd-Gen von Corynebacterium glutamicum Stamm ATCC 21529 ist beispielsweise in der US 6,927,046 beschrieben.

Das lysA-Gen von Corynebacterium glutamicum ATCC13869 (Brevibacterium lactofermentum) ist beispielsweise in der US 6,090,597 beschrieben.

Das ddh-Gen ist beispielsweise bei Ishino et al. (Agricultural and Biological Chemistry 52(11), 2903-2909 (1988)) beschrieben.

Das aat-Gen von Corynebacterium glutamicum ATCC13032 ist beispielsweise bei Kalinowski et al (Journal of Biotechnology 104 (1-3), 5-25 (2003); siehe auch Zugangsnummer NC_006958) beschrieben. Es wird dort als aspB-Gen bezeichnet. In der US 6,004,773 wird ein für eine Aspartat-Aminotransferase kodierendes Gen als aspC bezeichnet. Marienhagen et al (Journal of Bacteriology 187 (22), 7693-7646 (2005) bezeichnen das aat-Gen als aspT-Gen.

Das lysE-Gen von Corynebacterium glutamicum R127 ist beispielweise in der US 6,858,406 beschrieben. In gleicher Weise kann das in der US 6,861,246 verwendete lysE-Gen von Stamm ATCC13032 eingesetzt werden.

Das pyc-Gen von Corynebacterium glutamicum von Stamm ATCC 13032 ist beispielsweise in der WO 99/18228 und WO 00/39305 beschrieben. Weiterhin können Allele des pyc-Gens verwendet werden wie sie beispielsweise in der US 6,965,021 beschrieben sind. Die in dieser Patentschrift beschriebenen Pyruvat-Carboxylasen besitzen einen oder mehrere der Aminosäureaustausche ausgewählt aus der Gruppe: Pyc E153D (Austausch von L-Glutaminsäure an Position 153 gegen L-Asparaginsäure), Pyc A182S (Austausch von L-Alanin an Position 182 gegen L-Serin), Pyc A206S (Austausch von L-Alanin an Position 206 gegen L-Serin), Pyc H227R (Austausch von L-Histidin an Position 227 gegen L-Arginin), Pyc A455G (Austausch von L-Alanin an Position 455 gegen Glycin), und Pyc D1120E (Austausch von L-Asparaginsäure an Position 1120 gegen L-Glutaminsäure). In gleicher weise kann das in der EP 1 108 790 beschrieben pyc-Allel verwendet werden, das für eine Pyruvat-Carboxylase kodiert, welche den Aminosäureaustausch Pyc P458S (Austausch von L-Prolin an Position 458 gegen L-Serin) enthält.

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm. Unter einem Ausgangsstamm (Elternstamm) versteht man den Stamm, an dem die zur Überexpression führende Maßnahme durchgeführt wurde.

Die Erhöhung der Konzentration oder Aktivität lässt sich beispielsweise dadurch erzielen, dass man die Kopienzahl der entsprechenden Polynukleotide chromosomal oder extrachromosomal um mindestens eine Kopie erhöht.

Eine weit verbreitete Methode zur Erhöhung der Kopienzahl besteht darin, dass man das entsprechende Polynukleotid in einen Vektor, bevorzugt ein Plasmid, einbaut, der von einem coryneformen Bakterium repliziert wird. Weiterhin kann man als Vektoren Transposons, Insertionselemente (IS-Elemente) oder Phagen einsetzen Im Stand der Technik ist eine Fülle geeigneter Vektoren beschrieben.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines coryneformen Bakteriums eingefügt. Derartige Amplifikationsverfahren sind beispielsweise in der WO 03/014330 oder WO 03/040373 beschrieben.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in der Fig. 1 des Übersichtsartikel von Patek et al. (Journal of Biotechnology 104(1-3), 311-323 (2003)) beschrieben. In gleicher Weise können die von Vasicova et al (Journal of Bacteriology 181, 6188-6191 (1999)) beschrieben Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleobasen vom Startkodon eingefügt werden.

Durch die Maßnahmen der Überexpression, wird die Aktivität oder Konzentration des entsprechenden Polypeptids im allgemeinen um mindestens 10%, 25%, 50%, 75%, 100%, 150%, 200%, 300%, 400% oder 500%, maximal bis 1000% oder 2000% bezogen auf die Aktivität oder Konzentration des Polypeptids im Stamm vor der zur Überexpression führenden Maßnahme, erhöht.

Es ist ebenfalls möglich, zusätzlich zu den Maßnahmen die das amtR-Gen betreffen, einzelne Biosynthesegene abzuschwächen oder auszuschalten.

So ist es gegebenenfalls zweckmäßig für die Verbesserung der Produktion von L-Lysin, L-Valin oder L-Isoleucin, bevorzugt L-Lysin, eines oder mehrere der Gene ausgewählt aus der Gruppe
a) ein für die Glucose-6-Phosphat-Isomerase (Pgi, EC Nr. 5.3.1.9) kodierendes Gen pgi, wie beispielsweise das in der US 6,586,214 und US 6,465,238 beschriebene pgi-Gen von Corynebacterium glutamicum,
b) ein für die Malat-Dehydrogenase (Mdh, EC Nr. 1.1.1.37) kodierendes Gen mdh, wie beispielsweise in der WO 02/02778 beschrieben,
c) ein für die Malat-Chinon Oxidoreduktase (Mqo, EC Nr. 1.1.99.16) kodierendes Gen mqo, wie beispielsweise in der US 7,094,106 und PCT/EP2005/057216 beschrieben, und
d) ein für die E1p Untereinheit des Pyruvat-Dehydrogenase Komplexes kodierendes aceE-Gen (AceE, EC Nr. 1.2.4.1), wie beispielsweise in der EP-A-1767616 beschrieben,
abzuschwächen oder auszuschalten.

Diese Massnahmen können im Falle der L-Lysin Produktion auch zusätzlich zur Verwendung von lysC^{FBR}-Allelen und/oder der Überexpression eines oder mehrerer Gene ausgewählt aus der Gruppe dapA, dapB, asd, ddh, lysA, aat, lysE und pyc durchgeführt werden.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Im Falle des AceE-Polypeptides (siehe SEQ ID NO:2 von EP-A-1767616) kann die Abschwächung auch durch einen oder mehrere der Aminosäureaustausche ausgewählt aus der Gruppe a) Austausch von Ala an Position 225 gegen Val, Leu oder Ile, bevorzugt Val, b) Austausch von Gly an Position 255 gegen Ser oder Thr, bevorzugt Ser, c) Austausch von Asn an Position 282 gegen Gln, und d) Austausch des Cys an Position 283 gegen eine andere Aminosäure, bevorzugt Ser, erzielt werden, wobei folgende Austausche ausgewählt aus der Gruppe e) Austausch an Position 282, f) gleichzeitiger Austausch an den Positionen 225 und 283, und g) gleichzeitiger Austausch an den Positionen 255 und 283, bevorzugt werden.

Die Konzentration eines Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2476-2492 (1999)) bestimmt werden. Die Aktivität kann mit Hilfe eines geeigneten Enzymtest bestimmt werden.

Bei dem AmtR-Regulator, der im Folgenden auch als AmtR-Polypeptid oder Transkriptionsregulator AmtR bezeichnet wird, handelt es sich um ein Polypeptid mit der Aktivität eines Transkriptionsregulators, der bei Stickstoffüberschuss in der Zelle die Expression der Gene des Stickstoffstoffwechsels reprimiert. Bei Stickstoffmangel erfolgt die Derepression.

Bei Kultur einer Wildtypstammes eines coryneformen Bakteriums, bevorzugt Corynebacterium glutamicum wie beispielsweise Stamm ATCC 13032, in einem Minimalmedium, welches als Stickstoffquelle Ammonium-Ionen enthält, besteht Stickstoffmangel bei einer Ammonium-Ionen Konzentration von ≤ 5 mM, bevorzugt ≤ 1 mM, besonders bevorzugt ≤ 0,5 mM.

Der Adenylierungsgrad des Signaltransduktionsproteins GlnK im Cytoplasma des coryneformen Bakteriums gibt ebenfalls Auskunft darüber, ob für die Zelle Stickstoffmangel oder Stickstoffüberschuss besteht. Stickstoffmangel liegt dann vor wenn in der Zelle ≥ 80%, bevorzugt ≥ 90%, ≥ besonders bevorzugt 95% des Signaltransduktionsproteins in adenylierter Form vorliegen.

Zu den Genen des Stickstoffwechsels, die vom AmtR-Regulator bei Stickstoffüberschuss reprimiert werden, zählen unter Anderem amtA, amtB, codA, crnT, gdh, glnA, gluA, gltB, ureA und urtA (Tabelle 1 auf Seite 584 bei Beckers et al. (Molecular Microbiology 58(2), 580-595 (2005)).

Die Aminosäuresequenz des AmtR-Regulators coryneformer Bakterien ist zu mindestens 85% oder zu mindestens 90%, bevorzugt zu mindestens 95%, besonders bevorzugt zu mindestens 98% oder zu mindestens 99% identisch mit der Aminosäuresequenz von SEQ ID NO:2 und umfasst oder besitzt im Wesentlichen eine Länge von 222 Aminosäuren, wobei eine Länge von 222 Aminosäuren bevorzugt wird. Ein Beispiel für einen AmtR-Regulator der mindestens 98% identisch ist mit der Aminosäuresequenz von SEQ ID NO:2 ist der von Stamm ATCC14067. Er ist in SEQ ID NO:21 aufgerührt. Ganz besonders bevorzugt umfasst oder besitzt der AmtR-Regulator die Aminosäuresequenz von SEQ ID NO:2. Gegebenenfalls enthält die Aminosäuresequenz von SEQ ID NO:2 oder 21, maximal 3, bevorzugt maximal 2, besonders bevorzugt maximal einen konservative(n) Aminosäureaustausch(e). Durch die konservativen Aminosäureaustausche wird die Aktivität des AmtR-Repressors im Wesentlichen nicht verändert.

Der Begriff "im Wesentlichen eine Länge von 222 Aminosäuren" trägt in diesem Zusammenhang der Tatsache Rechnung, dass durch Insertion oder Deletion einer (1) oder mehrerer, maximal 10, 9, 8, 7, 6, 5, 4, 3 oder 2, Aminosäuren innerhalb des Polypeptids oder am N- oder C-terminalen Ende des Polypeptids die Länge des kodierten Polypeptids bei verschiedenen Arten oder Stämmen L-Aminosäure auscheidender coryneformer Bakterien geringfügig variiert. Ein Beispiel hierfür ist der AmtR-Regulator von Corynebacterium efficiens. Die Länge des Polypeptids (Siehe SEQ ID NO:11) beträgt in diesem Fall 223 Aminosäuren. Die unterschiedliche Länge des AmtR-Regulators ist durch Insertion der Aminosäure L-Glutaminsäure zwischen Position 151 und 153 von SEQ ID NO:2 bedingt.

Nachfolgend sind die Aminosäuren der Aminosäuresequenz des AmtR-Regulators von Corynebacterium efficiens (AmtR Ceff) den Aminosäuren der Aminosäuresequenz des AmtR-Regulators von Corynebacterium glutamicum (AmtR Cglu) zugeordnet.

| | | |
|---|---|---|
| AmtR Ceff | 1 | magavgrprrsaprragknpreeildasaelftrqgfattsthqiadavg |
| AmtR Cglu | 1 | magavgrprrsaprragknpreeildasaelftrqgfattsthqiadavg |
| | | |
| AmtR Ceff | 51 | irqaslyyhfpskteifltllkstvepsmvlagdlanleaspelrlwalv |
| AmtR Cglu | 51 | irqaslyyhfpskteifltllkstvepstvlaedlstldagpemrlwaiv |
| | | |
| AmtR Ceff | 101 | aaevrlllstkwnvgrlyqlpivaseefeeyhtqratltdtfrslateiv |
| AmtR Cglu | 101 | asevrlllstkwnvgrlyqlpivgseefaeyhsqrealtnvfrdlateiv |
| | | |
| AmtR Ceff | 151 | geddpraelpfhitmsaiemrrndgkvpsplsedslpdtavmladaalav |
| AmtR Cglu | 151 | g-ddpraelpfhitmsviemrrndgkipsplsadslpetaimladaslav |
| | | |
| AmtR Ceff | 201 | lgadlpgdrvertlellrqadak |
| AmtR Cglu | 200 | lgaplpadrvektlelikqadak |

Für die Zuordnung der einzelnen Aminosäuren verschiedener AmtR-Regulatoren können auch sogenannte Alignment-Programme, wie beispielsweise das ClustalW- (Thompson et al., Nucleic Acids Research 25(24), 4876-82 (1997)) oder MAFFT-Progamm (Katoh et al., Nucleic Acid Res., 30:3059-3066 (2002)) verwendet werden. Die einzelnen Aminosäuren eines Polypeptids können dadurch, trotz formal unterschiedlicher Länge der Aminosäuresequenzen, einander eindeutig zugeordnet werden.

Bei den aromatischen L-Aminosäuren spricht man von konservativen Austauschen wenn L-Phenylalanin, L-Tryptophan und L-Tyrosin gegeneinander ausgetauscht werden. Bei den hydrophoben L-Aminosäuren spricht man von konservativen Austauschen wenn L-Leucin, L-Isoleucin und L-Valin gegeneinander ausgetauscht werden. Bei den polaren Aminosäuren spricht man von konservativen Austauschen wenn L-Glutamin und L-Asparagin gegeneinander ausgetauscht werden. Bei den basischen Aminosäuren spricht man von konservativen Austauschen wenn L-Arginin, L-Lysin und L-Histidin gegeneinander ausgetauscht werden. Bei den sauren L-Aminosäuren spricht man von konservativen Austauschen wenn L-Asparaginsäure und L-Glutaminsäure gegeneinander ausgetauscht werden. Bei den Hydroxyl-Gruppen enthaltenden Aminosäuren spricht man von konservativen Austauschen wenn L-Serin und L-Threonin gegeneinander ausgetauscht werden.

Der Ausdruck "wird die Aktivität des AmtR-Repressors im Wesentlichen nicht verändert" bedeutet, dass durch die genannten maximal 3 konservativen Aminosäureaustausche, die Fähigkeit des AmtR-Repressors an seine DNA-Bindestelle zu binden um maximal 10 %, bevorzugt maximal 5 %, besonders bevorzugt 1 % und ganz besonders bevorzugt nicht verändert wird. Die Aktivität kann durch Verzögerungsgelelektrophorese (gel retardation assay) unter Verwendung von doppelsträngigen DNA-Molekülen mit der Nukleotidsequenz einer AmtR-Bindestelle des amtA-Gens (Siehe SEQ ID NO:14 und 15), einer AmtR-Bindestelle des amtB-Gens (Siehe SEQ ID NO:16 und 17) oder einer AmtR-Bindestelle des gltB-Gens (Siehe SEQ ID NO:18 und 19), bevorzugt unter Verwendung von doppelsträngigen DNA-Molekülen mit der Nukleotidsequenz einer AmtR-Bindestelle des amtA-Gens und ganz besonders bevorzugt unter Verwendung eines doppelsträngigen DNA-Molekülen mit der Nukleotidsequenz von SEQ ID NO:14 gemessen werden. Die Nukleotidsequenzen sind der Tabelle 1 von Beckers et al. (Molecular Microbiology 58(2), 580-595 (2005)) entnommen.

In der SEQ ID NO: 1 ist die Nukleotidsequenz der Kodierregion des amtR-Gens vom Typstamm von Corynebacterium glutamicum (Wildtypgen), das heißt ATCC13032, gemäß der Angaben der Datenbank des National Center for Biotechnology Information (NCBI) wiedergegeben. SEQ ID NO:2 und 4 zeigen die Aminosäuresequenz des kodierten Polypeptids. SEQ ID NO:2 und 4 enthalten an Position 34 L-Arginin, an Position 87 L-Threonin und an Position 203 L-Prolin. Es ist bekannt, dass durch wirtseigene Enzyme, sogenannte Aminopeptidasen, das endständige Methionin bei der Proteinsynthese entfernt werden kann. In der SEQ ID NO:3 sind stromaufwärts (upstream) und stromabwärts (downstream) gelegene Nukleotidsequenzen zusätzlich angegeben.

In der SEQ ID NO: 10 ist die Nukleotidsequenz der Kodierregion des amtR-Gens von Corynebacterium efficiens Stamm YS-314 gemäß der Angaben der Datenbank des National Center for Biotechnology Information (NCBI) wiedergegeben. SEQ ID NO:11 zeigt die Aminosäuresequenz des kodierten Polypeptids.

In der SEQ ID NO: 20 ist die Nukleotidsequenz der Kodierregion des amtR-Gens von Corynebacterium glutamicum ATCC14067 wiedergegeben. Die Sequenz wurde vom Anmelder bestimmt. SEQ ID NO:21 zeigt die Aminosäuresequenz des kodierten Polypeptids. Die Aminosäuresequenz von SEQ ID NO: 21 enthält an Position 34 L-Histidin, an Position 87 L-Isoleucin und an Position 203 L-Serin.

Die Nukleotidsequenz des Genoms von Corynebacterium glutamicum wurde von verschiedenen Arbeitsgruppen bestimmt.

Die von Kalinowski et al. (Journal of Biotechnology 104(1-3), 5-25 (2003)) von der Universität Bielefeld (Deutschland) bestimmte Sequenz von Stamm ATCC13032 ist unter der Zugangsnummer NC_006985 verfügbar. Dem amtR-Gen wird dort die Bezeichnung cg0986 zugeordnet und umfasst die Region von Position 923864-924532 des komplementären Stranges.

Die von Ikeda und Nakagawa (Applied Microbiology 62(2-3), 99-109 (2003)) von der Kitasato Universität (Japan) bestimmte Sequenz von Stamm ATCC13032 ist unter der Zugangsnummer NC_003450 verfügbar. Dem amtR-Gen wird dort die Bezeichnung NCgl0828 zugeordnet.

Die von Yukawa et al. (Microbiology 153(Pt 4),1042-58 (2007)) vom Research Institute of Innovative Technology for the Earth (RITE) (Japan) bestimte Sequenz von Stamm R ist unter der Zugangsnummer NC_009342 verfügbar. Dem amtR-Gen wird dort die Bezeichnung cgR_0978 zugeordnet.

Die Nukleotidsequenz des Genoms von Corynebacterium efficiens wurde von Nishio et al. (Genome Research 2003 13(7), 1572-1579 (2003)) von der Firma Ajinomoto (Japan) bestimmt. Sie ist unter der Zugangsnummer NC_004369 verfügbar. Dem amtR-Gen wird dort die Bezeichnung COG1309K zugeordnet und umfasst die Region von Position 1002436-1003107 des komplementären Stranges.

Der AmtR-Regulator gehört zur TetR-Familie der Transkriptionsregulatoren und weist wie die anderen Mitglieder dieser Familie an der DNA-Bindestelle ein typisches Helix-Turn-Helix Motiv auf (Ramos et al., Microbiology and Molecular Biology Reviews 69(2): 326-356 (2003); Jacoby et al., Molecular Microbiology 37(4): 964-977 (2000)).

Die Nukleotidsequenzen der DNA, an die der AmtR-Regulator bindet, sind bekannt. Von Jakoby et al. (Molecular Microbiology 37(4), 964-977 (2000)) wurde mittels Deletionsanalysen, Verzögerungsgelelektrophorese (gel retardation assay) und dem Matchmaker One-Hybrid-System (Clontech Laboratories, Inc., Mountain View, USA) die Expression des amt-Gens, das für den Ammonium-Transporter Amt in Corynebacterium glutamicum (Siewe et al., Journal of Biological Chemistry 271 (10): 5398-5402 (1996)) kodiert, untersucht. Der Ammonium-Transporter Amt wird bei Jakoby et al. auch als "(methyl)ammonium uptake system" bezeichnet. Bei Beckers et al. (Molecular Microbiology 58(2), 580-595 (2005)) wird das amt-Gen als amtA-Gen bezeichnet. Es trägt die NCBI Zugangsnummer NCgl1521. Von Jakoby et al. wurde gezeigt, dass die Expression des amt-Gens durch Bindung des AmtR-Regulators an Bindemotive doppelsträngiger DNA mit der Nukleotidsequenz 5'-ATCTATAGAACGATAG-3' und 5'-ATCTATAGGCGGATAG-3' reprimiert wird.

Von Beckers et al. (Molecular Microbiology 58(2), 580-595 (2005)) wurde das Konsensus Motiv (consensus motif) der Bindestelle für die vom AmtR-Regulator regulierten Gene bestimmt. Beckers et al. geben im Gegensatz zu Jakoby et al. (Molecular Microbiology 37(4), 964-977 (2000)) die Nukleotidsequenz des revers komplementären DNA-Stranges der Bindestelle an.

Weitere Ausführungen zum AmtR-Regulator findet man unter anderem bei Walter et al. (Journal of Molecular Microbiology 12, 131-138 (2007)) und A. Burkovski (Archives of Microbiology 179: 83-88 (2003); Aufsatz "Nitrogen Metabolism and its Regulation" im "Handbook of Corynebacterium glutamicum" (Eds.: L. Eggeling und M. Bott, CRC Press, Taylor & Francis, 2005).

Der Begriff "Abschwächung" beinhaltet die Verringerung der intrazellulären Konzentration oder Aktivität eines oder mehrerer Polypeptide (Proteine) bzw. Enzyme in einem Mikroorganismus, die durch die entsprechende DNA kodiert werden, im Vergleich zum Elternstamm. Als Elternstamm oder Ausgangsstamm wird der Stamm bezeichnet, an dem die Maßnahmen der Abschwächung durchgeführt wurden. Die Abschwächung kann erzielt werden, indem die Expression eines Polypeptids, beispielsweise durch Verwendung eines schwachen Promotors, verringert wird oder indem ein Allel verwendet wird, das für ein Polypeptid mit einer niedrigeren Aktivität kodiert und gegebenenfalls diese Maßnahmen inaktiviert.

Die Promotorregion des amtR-Gens ist in SEQ,ID NO:5 dargestellt. Die Nukleotidsequenz von SEQ ID NO:5 ist in SEQ ID NO:3 enthalten. Position 1 von SEQ ID NO:5 entspricht Position 911 von SEQ ID NO:3. Position 90 von SEQ ID NO:5 entspricht Position 1000 von SEQ ID NO:3.

Durch die Verringerung der Expression des amtR-Gens wird die intrazelluläre Konzentration des AmtR-Regulators auf >0 % bis ≤75 %, >0 % bis ≤50 %, >0 % bis ≤25 %, >0 % bis ≤ 5 %,>0 % bis ≤ 1 %, oder auf ≥ 0,1 % bis ≤75 %, ≥ 0,1 % bis ≤50 %, ≥ 0,1 % bis ≤ 25 %, ≥ 0,1 % bis ≤ 5 %, ≥ 0,1 % bis ≤ 1 %, oder auf ≥ 1 % bis ≤75 %, ≥ 1 % bis ≤50 %, ≥ 1 % bis ≤ 25 %, ≥ 1 % bis ≤ 5 %, oder auf ≥ 5 % bis ≤75 %, ≥ 5 % bis ≤50 %, ≥ 5 % bis ≤ 25 % der Konzentration im Elternstamm bzw. Ausgangsstamm herabgesetzt.

Erfindungsgemäß wird schließlich durch einen Austausch des Glycin an Position 3 der Aminosäuresequenz, bevorzugt der Aminosäuresequenz gemäß SEQ ID NO:2, SEQ ID NO:21 oder SEQ ID NO:11, gegen L-Glutaminsäure die Aktivität des AmtR-Regulators verringert.

Durch den erfindungsgemäßen Aminosäureaustausch wird die Aktivität des AmtR-Regulators auf > 0 % bis ≤ 75 %, > 0 % bis ≤50 %, >0 % bis ≤ 25 %, > 0 % bis ≤ 5 %,> 0 % bis ≤ 1 %, oder auf ≥ 0,1 % bis ≤75 %, ≥ 0,1 % bis ≤50 %, ≥ 0,1 % bis ≤ 25 %, ≥ 0,1 % bis ≤ 5 %, ≥ 0,1 % bis ≤ 1 %, oder auf ≥ 1 % bis ≤75 %, ≥ 1 % bis ≤50 %, ≥ 1 % bis ≤ 25 %, ≥ 1 % bis ≤ 5 % oder auf ≥ 5 % bis ≤75 %, ≥ 5 % bis ≤50 %, ≥ 5 % bis ≤ 25 % der Aktivität des AmtR-Regulators des Wildtyps, bevorzugt mit der Aminosäuresequenz von SEQ ID NO:2 oder SEQ ID NO:11 verringert.

Darüberhinaus ist es möglich die Expression der erfindungsgemäßen Varianten des AmtR-Regulators durch Verwendung bekannter, schwacher Promotoren abzusenken bzw. einzustellen. Hierzu gehören unter anderem die Promotoren seqP-RBS_01 to seqP-RBS_07, die in der Zeitschrift Research Disclosure unter der Nummer 512057 (Ausgabe Dezember 2006) offengelegt sind und die von M. Patek beschriebenen Varianten des dapA-Promotors, bevorzugt die Varianten C7, C13, O1, C2, J2, B31, C5 und B6 (M. Patek im "Handbook of Corynebacterium glutamicum" (Lothar Eggeling and Michael Bott (editors), CRC Press, Taylor and Francis Group, Boca Raton, FL, USA, 2005)).

Die Abschwächung des amtR-Gens kann mit verschiedenen Methoden bestimmt werden. Die Konzentration ist mit Hilfe von 1- und 2-dimensionaler Proteingelauftrennung und anschließender Bestimmung der Proteinkonzentration im Gel nachweisbar. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann weiterhin durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich (1999) Angewandte Chemie 111:2630-2647) analysiert werden. Die Aktivität des AmtR-Regulators als DNA-bindendem Protein kann mittels Verzögerungsgelelektrophorese (retardation gel electrophoresis) (Wilson et al. (2001) Journal of Bacteriology 183:2151-2155) gemessen werden. Dieser Test wird auch als "DNA band shift assay" bezeichnet. Die Wirkung von DNA-bindenden Proteinen auf die Expression der von ihnen kontrolierten Gene kann schließlich auch durch verschiedene, gut beschriebene Methoden des Reportergen-Assays nachgewiesen werden (Sambrook et al., Molecular cloning: a laboratory manual. 2nd Ed. Cold Spring Harbor Laboratory Press, Cold Spring Harbor, N.Y., 1989).

Gegenstand der Erfindung ist schließlich ein isoliertes Polynukleotid kodierend für einen AmtR-Regulator, dessen Aminosäuresequenz zu mindestens 90%, bevorzugt zu mindestens 95%, besonders bevorzugt zu mindestens 98% oder zu mindestens 99% und.ganz besonders bevorzugt identisch ist mit der Aminosäuresequenz von SEQ ID NO:2 und im Wesentlichen eine Länge von 222 Aminosäuren, bevorzugt eine Länge von 222 Aminosäuren, umfasst oder besitzt und einen (1) Aminoäureaustausch an der Position 3 aufweist, wobei das Glycin an Position 3 gegen L-Glutaminsäure ausgetauscht wird.

Bevorzugt wird ein isoliertes Polynukleotid, das für einen AmtR-Regulator kodiert, der die Aminosäuresequenz von SEQ ID NO:2 umfasst oder besitzt und der einen (1) Aminosäureaustausch an der Position 3 aufweist, wobei das Glycin an Position 3 gegen L-Glutaminsäure ausgetauscht wird.

Gegebenenfalls enthält die Aminosäuresequenz darüber hinaus maximal 3, bevorzugt maximal 2 besonders bevorzugt maximal (einen) konservative(n) Aminosäureaustausch(e).

Bevorzugt wird weiterhin ein isoliertes Polynukleotid, das für einen AmtR-Regulator kodiert, der die Aminosäuresequenz von SEQ ID NO:11 umfasst oder besitzt und der einen (1) Aminosäureaustausch an der Position 3 aufweist, wobei das Glycin an Position 3 gegen L-Glutaminsäure ausgetauscht wird. Gegebenenfalls enthält die Aminosäuresequenz darüber hinaus maximal 3, bevorzugt maximal 2 besonders bevorzugt maximal (einen) konservative(n) Aminosäureaustausch(e).

Bevorzugt wird schließlich ein isoliertes Polynukleotid, das für einen AmtR-Regulator kodiert, der die Aminosäuresequenz von SEQ ID NO:21 umfasst oder besitzt und der einen (1) Aminosäureaustausch an der Position 3 aufweist, wobei das Glycin an Position 3 gegen L-Glutaminsäure ausgetauscht wird. Gegebenenfalls enthält die Aminosäuresequenz bevorzugt maximal (einen) konservative(n) Aminosäureaustausch(e).

Ein Beispiel für einen konservativen Aminosäureaustausch ist der Austausch des Valin an Position 141 der Aminosäuresequenz gegen Isoleucin.

Besonders bevorzugt wird ein isoliertes Polynukleotid, das für einen AmtR-Regulator mit der Aminosäuresequenz von SEQ ID NO:2, kodiert, wobei das Glycin an Position 3 gegen L-Glutaminsäure ausgetauscht wird. Die Aminsäuresequenz der Variante des AmtR-Polypeptids welche L-Glutaminsäure an Position 3 enthält ist in SEQ ID NO:6 und 8 dargestellt.

Gegenstand der Erfindung ist weiterhin ein isoliertes Polynukleotid, welches die Nukleotidsequenz gemäß SEQ ID NO:5 oder 7 umfasst oder besitzt.

Gegenstand der Erfindung sind auch Vektoren, welche die erfindungsgemäßen Polynukleotide enthalten.

Gegenstand der Erfindung sind schließlich auch Zellen von Mikroorganismen, insbesondere von Bakterien, bevorzugt der Gattung Corynebacterium und Escherichia, besonders bevorzugt der Art Corynebacterium glutamicum und Escherichia coli, die die genannten Polynukleotide oder Vektoren enthalten oder unter Verwendung der genannten Polynukleotide oder Vektoren hergestellt wurden.

Zur Herstellung der erfindungsgemäßen Polynukleotide können klassische in-vivo Mutageneseverfahren mit Zellpopulationen von Bakterien der Gattung Corynebacterium unter Verwendung mutagener Stoffe wie beispielsweise N-Methyl-N'-Nitro-N-Nitrosoguanidin (MNNG) oder von ultraviolettem Licht verwendet werden. Anschließend wird aus den Mutanten DNA bereitgestellt, beziehungsweise isoliert und mit Hilfe der Polymerase-Kettenreaktion (PCR) unter Verwendung von Primerpaaren, die die Amplifizierung des amtR-Gens bzw. amtR-Allels erlauben, das entsprechende Polynukleotid synthetisiert und isoliert. Hierzu können beliebige Primerpaare aus der stromaufwärts und stromabwärts der Kodierregion gelegenen Nukleotidsequenz und der dazu komplementären Nukleotidsequenz ausgewählt werden

Anleitungen und Informationen zur PCR findet der Fachmann beispielsweise im Handbuch "PCR-Strategies" (Innis, Felfand und Sninsky, Academic Press, Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994). Weitere Anleitungen zur PCR finden sich beispielsweise in der WO 06/100177 auf Seiten 15 bis 17.

In einem weiteren Arbeitschritt wird dann die Nukleotidsequenz des Polynukleotids bestimmt. Diese kann beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067 (1990)) angegebenen Modifikationen bestimmt werden.

Das von dieser Nukleotidsequenz kodierte Polypeptid kann dann bezüglich der Aminosäuresequenz analysiert werden. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Es ist ebenfalls möglich das erfindungsgemäße Polynukleotid bzw. amtR-Allel, durch Methoden der in-vitro Genetik herzustellen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992) oder der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA). Weiterhin können mutagene Oligonukleotide, wie bei T. A. Brown (Gentechnologie für Einsteiger, Spektrum Akademischer Verlag, Heidelberg, 1993) und R. M. Horton (PCR-Mediated Recombination and Mutagenesis, Molecular Biotechnology 3, 93-99 (1995)) beschrieben, eingesetzt werden. Die von Papworth et al. (Strategies 9(3), 3-4 (1996)) beschriebene Methode unter Verwendung des "Quik Change Site-directed Mutagenesis Kit" der Firma Stratagene (La Jolla, California, USA) kann ebenfalls eingesetzt werden.

Die auf diese Weise hergestellten Polynukleotide, können dazu verwendet werden, um rekombinante Stämme der Gattung Corynebacterium, bevorzugt Corynebacterium glutamicum herzustellen, die die erfindungsgemäßen Varianten des AmtR-Regulators enthalten und/oder die erfindungsgemäßen Modifikationen im Promotorbereich des amtR-Gens enthalten und die im Vergleich zum Ausgangs- beziehungsweise Elternstamm in erhöhtem Umfang L-Aminosäuren in das sie umgebende Medium abgeben und/oder im Zellinneren anhäufen.

Eine verbreitete Methode zum Einbau von Mutationen in Gene von Bakterien der Gattung Corynebacterium, insbesondere der Art Corynebacterium glutamicum, ist die des Allelaustausches, die auch unter der Bezeichnung "gene replacement" bekannt ist. Bei diesem Verfahren wird ein DNA-Fragment, welches die interessierende Mutation enthält, in den gewünschten Stamm überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die mutierte Sequenz ausgetauscht.

Das DNA-Fragment, enthaltend die interessierende Mutation, liegt bei dieser Methode typischerweise in einem Vektor, insbesondere einem Plasmid, vor, das vorzugsweise von dem mit der Mutation zu versehenden Stamm nicht oder nur begrenzt, d. h. unter ausgewählten Kulturbedingungen, repliziert wird. Als Hilfs- oder Zwischenwirt, in dem der Vektor replizierbar ist, wird im Allgemeinen ein Bakterium der Gattung Escherichia, bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in Corynebacterium replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat. Diese ermöglichen die Selektion auf Rekombinationsereignisse, bei denen der Vektor aus dem Chromosom eliminiert wird.

Weiterhin wurde von Nakamura et al. (US 6,303,383) ein Temperatur-sensitives Plasmid für Corynebacterium beschrieben, das lediglich bei Temperaturen unterhalb von 31°C replizieren kann. Es kann ebenfalls für die Massnahmen der Erfindung eingesetzt werden.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) in das Corynebacterium überführt. Gegebenenfalls kann die Überführung der DNA auch durch ballistische Methoden (z. B. Partikelbeschuss) erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium. Als Zielgen bezeichnet man das Gen, in dem der gewünschte Austausch erfolgen soll.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Von Schwarzer und Pühler (Bio/Technology 9, 84-87 (1991)) wurde diese Methode verwendet, um ein lysA-Allel, welches eine Deletion trug, und um ein lysA-Allel, welches eine Insertion trug, in das Chromosom von C. glutamicum anstelle des Wildtypgens einzubauen. Von Nakagawa et al. (EP 1108790) und Ohnishi et al. (Applied Microbiology and Biotechnology 58(2), 217-223 (2002)) wurde diese Methode eingesetzt, um verschiedene Mutationen ausgehend von den isolierten Allelen bzw. Polynukleotiden in das Chromosom von C. glutamicum einzubauen.

So kann beispielsweise für den Einbau der Mutation, die zum Aminosäureaustausch des Glycins gegen L-Glutaminsäure an Position 3 von SEQ ID NO:2 führt, bevorzugt wie in SEQ ID NO:6 und 8 dargestellt, ein Polynukleotid bzw. DNA-Fragment verwendet werden, das mindestens die Nukleotidsequenz von Position 958 bis 1058 von SEQ ID NO:8 umfasst. Dieses DNA-Fragment enthält die erfindungsgemäße Mutation und stromaufwärts und stromabwärts davon eine Nukleotidsequenz mit einer Länge von jeweils mindestens 50 Nukleobasen.

Bevorzugt werden DNA-Fragmente die stromaufwärts und stromabwärts der Mutation eine Nukleotidsequenz mit einer Länge von jeweils mindestens ca. 100, besonders bevorzugt jeweils mindestens ca. 250 Nukleobasen und ganz besonders bevorzugt jeweils mindestens ca. 500 Nukleobasen besitzen. Die maximale Länge der stromaufwärts und stromabwärts der Mutation gelegenen Nukleotidsequenz liegt im Allgemeinen bei ca. 500, ca. 750, ca. 1000, ca. 1500, ca. 2000, ca. 3000, ca. 4000 oder 5000 Nukleobasen. Die Gesamtlänge des für den Allelaustausch eingesetzten Polynukleotids beträgt dementsprechend maximal ca. 1000, maximal ca. 1500, maximal ca. 2000, maximal ca. 3000, maximal ca. 4000, maximal ca. 6000, maximal ca. 8000 oder 10000 Nukleobasen.

Die Leistung der erfindungsgemäßen Bakterien der Gattung Corynebacterium bzw. des Fermentationsprozesses unter Verwendung derselben bezüglich eines oder mehrerer der Parameter ausgewählt aus der Gruppe der L-Aminosäure-Konzentration (gebildete L-Aminosäure pro Volumen), der L-Aminosäure-Ausbeute (gebildete L-Aminosäure pro verbrauchter Kohlenstoff-Quelle), der L-Aminosäure-Bildung (gebildete L-Aminosäure pro Volumen und Zeit) und der spezifischen L-Aminosäure-Bildung (gebildete L-Aminosäure pro Zelltrockenmasse bzw. Biotrockenmasse und Zeit oder gebildete L-Aminosäure pro Zellprotein und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben erhöht.

Die erfindungsgemäß hergestellten Bakterien der Gattung Corynebacterium können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion der gewünschten L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrübenoder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch-Verfahren wird die Kultivierung solange fortgesetzt, bis sich ein Maximum der gewünschten L-Aminosäure, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung (Akkumulation) der L-Aminosäure im Fermentationsmedium und/oder in den Bakterienzellen.

Beispiele für geeignete Fermentationsmedien finden sich unter anderem in den Patentschriften 5,770,409, US 5,840,551 und US 5,990,350 oder US 5,275,940.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtaldialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtaldialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Gegenstand der Erfindung ist dementsprechend auch ein Verfahren zur Herstellung von L-Aminosäuren, bevorzugt L-Lysin, L-Glutaminsäure, L-Glutamin, L-Arginin, L-Prolin und L-Ornithin, besonders bevorzugt L-Lysin, dadurch gekennzeichnet, dass man folgende Schritte durchführt:
a) Fermentation der erfindungsgemäßen coryneformen Bakterien, bevorzugt der Gattung Corynebacterium, besonders bevorzugt der Art Corynebacterium glutamicum, in einem geeignetem Nährmedium, und
b) Akkumulation der L-Aminosäure in dem Nährmedium und/oder in den Zellen der genannten Bakterien.

Anschließend erfolgt die Bereitstellung bzw. Herstellung oder Gewinnung eines L-Aminosäure haltigen Produktes in flüssiger oder fester Form.

Durch die Maßnahmen der Fermentation erhält man eine Fermentationsbrühe, welche die gewünschte L-Aminosäure enthält.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten L-Aminosäure sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) die im Laufe der Fermentation gebildete L-Aminosäure,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der jeweiligen L-Aminosäure erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein L-Aminosäure haltiges Produkt in flüssiger oder fester Form bereitzustellen. Hierfür wird auch der Ausdruck "Gewinnen des L-Aminosäure haltigen Produktes" verwendet. Im einfachsten Fall stellt die L-Aminosäure-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige ≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige ≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der L-Aminosäure. Auf diese Weise werden Produkte isoliert, die einen gewünschten Gehalt an L-Aminosäure aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

Durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen der L-Aminosäuren. Für die Massnahmen gemäß a), b), c) oder d) sind im Stand der Technik eine Fülle von technischen Anleitungen verfügbar.

Im Falle der Aminosäure L-Lysin sind im Stand der Technik im wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen aus der Fermentationsbrühe das L-Lysin-haltige Produkt oder das gereinigte L-Lysin hergestellt wird.

Zur Herstellung von festem, reinen L-Lysin werden im wesentlichen Methoden der Ionenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄).

In der EP-B-0534865 ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust (maximal 5%) der jeweiligen Aminosäure auftritt.

Ein weiterer Gegenstand der Erfindung ist ein Verfahren, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man ein Verfahren durchführt das mindestens folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(n) und
b) das so erhaltene Gemisch durch Wasserentzug aufkonzentriert, und gegebenenfalls granuliert,
   wobei gegebenenfalls vor Schritt a) eine oder beide der folgenden Massnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltigen Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²⁻ Anion, bzw. die Schwefelsäure zweiwertig ist. Ein Verhältnis V = 1 bedeutet, dass ein stöchiometrisch zusammengesetztes Lys₂-H₂SO₄) vorliegt, während bei einem Verhältnis von V = 0,9 ein 10%iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.-%, besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten L-Lysin Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Ein weiterer Gegenstand der Erfindung ist ein Verfahren zur Herstellung eines festen Lysin-haltigen Produktes, wie es in seinen Grundzügen in der US 20050220933 beschrieben ist, und das die Aufarbeitung der unter Verwendung der erfindungsgemäßen Mikroorganismen erhaltenen Fermentationsbrühe in folgenden Schritten umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Massnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel

2x[SO₄²⁻] + [Cl⁻] - [NH₄⁺] - [Na⁺] - [K⁺] - 2x[Mg²⁺] - 2x[Ca²⁺] / [L-Lys]

0,68 bis 0,95, bevorzugt 0,68 bis 0,90, besonders bevorzugt 0,68 bis 0,86 ergibt, so wie von Kushiki et al. in der US 20030152633 beschrieben.

Im Falle des L-Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

## Patentansprüche

1. Rekombinantes, L-Aminosäure ausscheidendes, coryneformes Bakterium bei dem das amtR-Gen, das für einen AmtR-Regulator kodiert, dessen Aminosäuresequenz mindestens 90% identisch ist mit der Aminosäuresequenz von SEQ ID NO:2 und im Wesentlichen eine Länge von 222 Aminosäuren umfasst, abgeschwächt worden ist, durch.

2. Bakterium gemäß Anspruch 1, **dadurch gekennzeichnet, dass** die Aminosäuresequenz des AmtR-Regulators eine Länge von 222 oder 223 Aminosäuren umfasst.

3. Bakterium gemäß Anspruch 2, **dadurch gekennzeichnet, dass** die Aminosäuresequenz des AmtR-Regulators die Sequenz von SEQ ID NO:2, SEQ ID NO:21 oder SEQ ID NO:11 umfasst.

4. Bakterium gemäß Anspruch 1, **dadurch gekennzeichnet**, das der Austausch des Glycin an Position 3 von SEQ ID NO:2 gegen L-Glutaminsäure durch den Austausch der Nukleobase Guanin an Position 8 von SEQ ID NO:1 gegen Adenin erzielt wird.

5. Bakterium gemäß einem der Ansprüche 1 bis 4, **dadurch gekennzeichnet, dass** das L-Aminosäure ausscheidendem Bakterium L-Lysin, L-Glutaminsäure, L-Glutamin, L-Arginin, L-Prolin oder L-Ornithin, bevorzugt L-Lysin ausscheidet.

6. Bakterium gemäß Anspruche 5, **dadurch gekennzeichnet, dass** das Bakterium im Falle eines L-Lysin ausscheidenden Bakteriums ein Polynukleotid enthält, das für ein Polypeptid mit Aspartatkinase-Aktivität kodiert, welche im Vergleich zum Wildtyp gegenüber der Hemmung durch Lysin und Threonin desensibilisiert ist.

7. Bakterium gemäß Anspruch 6, **dadurch gekennzeichnet, dass** das Polynukleotid, das für ein Polypeptid mit Aspartatkinase-Aktivität kodiert, überexprimiert wird.

8. Bakterium gemäß einem der Ansprüche 5-7, **dadurch gekennzeichnet, dass** das Bakterium im Falle eines L-Lysin ausscheidenden Bakteriums zusätzlich eines oder mehrere der Merkmale ausgewählt aus der folgenden Gruppe besitzt:
a) überexprimiertes Polynukleotid, das für eine Dihydrodipicolinat-Synthase (DapA)kodiert,
b) überexprimiertes Polynukleotid, das für eine Aspartatsemialdehyd-Dehydrogenase (Asd) kodiert,
c)überexprimiertes Polynukleotid, das für eine meso-Diaminopimelat-Dehydrogenase (Ddh) kodiert,
d) überexprimiertes Polynukleotid, das für eine Diaminopimelat-Decarboxylase (LysA) kodiert,
e)überexprimiertes Polynukleotid, das für eine Aspartat-Aminotransferase (Aat) kodiert,
f) überexprimiertes Polynukleotid, das für ein Polypeptid mit L-Lysin-Export Aktivität (LysE) kodiert,
g)überexprimiertes Polynukleotid, das für eine Pyruvat-Carboxylase (Pyc) kodiert, und
h) überexprimiertes Polynukleotid, das für eine Dihydrodipicolinat-Reduktase (DapB) kodiert.

9. Bakterium gemäß einem der Ansprüche 1 bis 8, **dadurch gekennzeichnet, dass** es sich bei dem coryneformen Bakterium um ein Bakterium der Gattung Corynebacterium, bevorzugt
um ein Bakterium der Art Corynebacterium glutamicum handelt.

10. Verfahren zur fermentativen Herstellung von L-Aminosäuren, **dadurch gekennzeichnet, dass** man folgende Schritte durchführt,
a) Fermentation eines Bakteriums gemäß einem der Ansprüche 1 bis 9 in einem Nährmedium, und
b) Akkumulation der L- Aminosäure in dem Nährmedium und/oder in den Zellen der genannten Bakterien.

11. Verfahren nach Anspruch 10 **dadurch gekennzeichnet, dass** es sich um ein Verfahren ausgewählt aus der Gruppe Satzverfahren, Zulaufverfahren und kontinuierliches Verfahren handelt.

12. Verfahren nach einem der Ansprüche 10 oder 11 **dadurch gekennzeichnet, dass** es sich bei der L-Aminosäure um L-Lysin handelt.

13. Verfahren nach einem der Ansprüche 10 -12, **dadurch gekennzeichnet, dass** man ein L-Aminosäure haltiges Produkt gewinnt.

14. Verfahren nach Anspruch 13, **dadurch gekennzeichnet, dass** man die L-Aminosäure reinigt oder,
dass Bestandteile der Fermentationsbrühe und/oder Biomasse in ihrer Gesamtheit oder in Anteilen (> 0 bis 100%) im Produkt verbleiben.

15. Verfahren nach einem der Ansprüche 10 bis 13, **dadurch gekennzeichnet, dass** man einer L-Lysin haltigen Fermentationsbrühe Wasser entzieht und ein Produkt mit einem Wassergehalt von maximal 5 Gew.-% erhält oder,
dass man eine L-Lysin haltige Fermentationsbrühe zunächst konzentriert und anschließend sprühtrocknet oder sprühgranuliert.

16. Isoliertes Polynukleotid kodierend für einen AmtR-Regulator, dessen Aminosäuresequenz zu mindestens 90% identisch ist mit der Aminosäuresequenz von SEQ ID NO:2 und im Wesentlichen eine Länge von 222 Aminosäuren umfasst und einen Aminosäureaustausche an der Position oder einer entsprechenden Position aufweist, wobei das Glyzin an dieser Position gegen L-Glutaminsäure ausgetauscht wird.

17. Vektor enthaltend ein Polynukleotid gemäß Anspruch 16.

18. Eine Mikroorganismen-Zelle enthaltend den Vektor gemäß Anspruch 17 oder ein Polynukleotid gemäß Anspruch 16.

## Claims

1. Recombinant, L-amino acid-secreting, coryneform bacterium in which the amtR gene which codes for an AmtR regulator whose amino acid sequence is at least 90% identical to the amino acid sequence of SEQ ID NO:2 and essentially comprises a length of 222 amino acids has been attenuated by exchange of the glycine at position 3 of the amino acid sequence for L-glutamic acid.

2. Bacterium according to Claim 1, **characterized in that** the amino acid sequence of the AmtR regulator includes a length of 222 or 223 amino acids.

3. Bacterium according to Claim 2, **characterized in that** the amino acid sequence of the AmtR regulator includes the sequence of SEQ ID NO:2, SEQ ID NO:21 or SEQ ID NO:11.

4. Bacterium according to Claim 1, **characterized in that** the exchange of the glycine at position 3 of SEQ ID NO:2 for L-glutamic acid is achieved by exchanging the nucleobase guanine at position 8 of SEQ ID NO:1 for adenine.

5. Bacterium according to any of Claims 1 to 4, **characterized in that** the L-amino acid-secreting bacterium secretes L-lysine, L-glutamic acid, L-glutamine, L-arginine, L-proline or L-ornithine, preferably L-lysine.

6. Bacterium according to Claim 5, **characterized in that** the bacterium in the case of an L-lysine-secreting bacterium comprises a polynucleotide which codes for a polypeptide having aspartate kinase activity which, by comparison with the wild type, is desensitized to inhibition by lysine and threonine.

7. Bacterium according to Claim 6, **characterized in that** the polynucleotide which codes for a polypeptide having aspartate kinase activity is overexpressed.

8. Bacterium according to any of Claims 5-7, **characterized in that** the bacterium in the case of an L-lysine-secreting bacterium additionally has one or more of the features selected from the following group:
a) overexpressed polynucleotide which codes for a dihydrodipicolinate synthase (DapA),
b) overexpressed polynucleotide which codes for an aspartate-semialdehyde dehydrogenase (Asd),
c) overexpressed polynucleotide which codes for a meso-diaminopimelate dehydrogenase (Ddh),
d) overexpressed polynucleotide which codes for a diaminopimelate decarboxylase (LysA),
e) overexpressed polynucleotide which codes for an aspartate aminotransferase (Aat),
f) overexpressed polynucleotide which codes for a polypeptide having L-lysine export activity (LysE),
g) overexpressed polynucleotide which codes for a pyruvate carboxylase (Pyc), and
h) overexpressed polynucleotide which codes for a dihydrodipicolinate reductase (DapB).

9. Bacterium according to any of Claims 1 to 8, **characterized in that** the coryneform bacterium is a bacterium of the genus Corynebacterium, preferably bacterium of the species Corynebacterium glutamicum.

10. Process for the fermentative production of L-amino acids, **characterized in that** it comprises carrying out the following steps,
a) fermentation of a bacterium according to any of Claims 1 to 9 in a nutrient medium, and
b) accumulation of the L-amino acid in the nutrient medium and/or in the cells of said bacteria.

11. Process according to Claim 10, **characterized in that** it is a process selected from the group of batch processes, fed batch processes and continuous processes.

12. Process according to either of Claims 10 or 11, **characterized in that** the L-amino acid is L-lysine.

13. Process according to any of Claims 10-12, **characterized in that** an L-amino acid-containing product is obtained.

14. Process according to Claim 13, **characterized in that** the L-amino acid is purified or **in that** constituents of the fermentation broth and/or biomass remain in their entirety or in portions (> 0 to 100%) in the product.

15. Process according to any of Claims 10 to 13, **characterized in that** water is removed from an L-lysine-containing fermentation broth, and a product with a water content not exceeding 5% by weight is obtained or **in that** an L-lysine-containing fermentation broth is initially concentrated and subsequently spray dried or spray granulated.

16. Isolated polynucleotide coding for an AmtR regulator whose amino acid sequence is at least 90% identical to the amino acid sequence of SEQ ID NO:2 and essentially comprises a length of 222 amino acids and includes an amino acid exchange at position 3 or a corresponding position, wherein the glycine at this position is exchanged for L-glutamic acid.

17. Vector comprising a polynucleotide according to Claim 16.

18. A microorganism cell comprising the vector according to Claim 17 or a polynucleotide according to Claim 16.

## Revendications

1. Bactérie coryneforme recombinante, excrétant un acide aminé L, chez laquelle le gène amtR, qui code pour un régulateur Amt-R dont la séquence d'acides aminés est identique à raison d'au moins 90 % à la séquence d'acides aminés de SEQ ID n° 2 et présente essentiellement une longueur de 222 acides aminés, a été affaibli par remplacement de la glycine à la position 3 de la séquence d'acides aminés par l'acide L-glutamique.

2. Bactérie selon la revendication 1, **caractérisée en ce que** la séquence d'acides aminés du régulateur AmtR présente une longueur de 222 ou 223 acides aminés.

3. Bactérie selon la revendication 2, **caractérisée en ce que** la séquence d'acides aminés du régulateur AmtR présente la séquence de SEQ ID n° 2, SEQ ID n° 21 ou SEQ ID n° 11.

4. Bactérie selon la revendication 1, **caractérisée en ce que** le remplacement de la glycine à la position 3 de SEQ ID n° 2 par l'acide L-glutamique est réalisé par remplacement de la base nucléique guanine à la position 8 de SEQ ID n° 1 par l'adénine.

5. Bactérie selon l'une quelconque des revendications 1 à 4, **caractérisée en ce que** la bactérie excrétant un acide aminé L excrète de la L-lysine, de l'acide L-glutamique, de la L-glutamine, de la L-arginine, de la L-proline ou de la L-ornithine, de préférence de la L-lysine.

6. Bactérie selon la revendication 5, **caractérisée en ce que** dans le cas d'une bactérie excrétant de la L-lysine la bactérie contient un polynucléotide qui code pour un polypeptide à activité aspartate kinase, qui par comparaison avec le type sauvage est désensibilisée vis-à-vis de l'inhibition par la lysine et la thréonine.

7. Bactérie selon la revendication 6, **caractérisée en ce que** le polynucléotide qui code pour un polypeptide à activité aspartate kinase est surexprimé.

8. Bactérie selon l'une quelconque des revendications 5 à 7, **caractérisée en ce que** dans le cas d'une bactérie excrétant de la L-lysine la bactérie possède en outre un ou plusieurs des caractères choisis dans le groupe suivant :
a) polynucléotide surexprimé, qui code pour une dihydrodipicolinate synthase (DapA),
b) polynucléotide surexprimé, qui code pour une aspartate-semialdéhyde déshydrogénase (Asd),
c) polynucléotide surexprimé, qui code pour une méso-diaminopimélate déshydrogénase (Ddh),
d) polynucléotide surexprimé, qui code pour une diaminopimélate décarboxylase (LysA),
e) polynucléotide surexprimé, qui code pour une aspartate aminotransférase (Aat),
f) polynucléotide surexprimé, qui code pour un polypeptide à activité L-lysine-export (LysE),
g) polynucléotide surexprimé, qui code pour une pyruvate carboxylase (Pyc), et
h) polynucléotide surexprimé, qui code pour une dihydrodipicolinate réductase (DapB),

9. Bactérie selon l'une quelconque des revendications 1 à 8, **caractérisée en ce que** la bactérie coryneforme consiste en une bactérie appartenant au genre *Corynebacterium,* de préférence en une bactérie appartenant à l'espèce *Corynebacterium glutamicum.*

10. Procédé pour la production d'acides L-aminés par fermentation, **caractérisé en ce qu'**on effectue les étapes suivantes,
a) culture par fermentation d'une bactérie selon l'une quelconque des revendications 1 à 9, dans un milieu nutritif, et
b) accumulation de l'acide aminé L dans le milieu nutritif et/ou dans les cellules desdites bactéries.

11. Procédé selon la revendication 10, **caractérisé en ce qu'**il s'agit d'un procédé choisi dans le groupe constitué par un procédé en mode discontinu, un procédé en mode discontinu avec additions et un procédé continu.

12. Procédé selon la revendication 10 ou 11, **caractérisé en ce que** l'acide aminé L consiste en L-lysine.

13. Procédé selon l'une quelconque des revendications 10 à 12, **caractérisé en ce qu'**on obtient un produit contenant un acide L-aminé.

14. Procédé selon la revendication 13, **caractérisé en ce qu'**on purifie l'acide L-aminé ou
**en ce que** les composants du bouillon de fermentation et/ou la biomasse restent en leur totalité ou en certaines proportions (> 0 à 100 %) dans le produit.

15. Procédé selon l'une quelconque des revendications 10 à 13, **caractérisé en ce qu'**on extrait l'eau d'un bouillon de fermentation contenant de la L-lysine et on obtient un produit ayant une teneur en eau d'au maximum 5 % en poids ou
**en ce que** d'abord on concentre un bouillon de fermentation contenant de la L-lysine et ensuite on le sèche par atomisation ou granule par pulvérisation.

16. Polynucléotide isolé codant pour un régulateur Amt-R dont la séquence d'acides aminés est identique à raison d'au moins 90 % à la séquence d'acides aminés de SEQ ID n° 2 et présente essentiellement une longueur de 222 acides aminés et comporte un remplacement d'acide aminé à la position 3 ou à une position correspondante, la glycine à cette position étant remplacée par l'acide L-glutamique.

17. Vecteur contenant un polynucléotide selon la revendication 16.

18. Cellule de micro-organisme contenant le vecteur selon la revendication 17 ou un polynucléotide selon la revendication 16.


### SEQUENZPROTOKOLL

<110> Evonik-Degussa GmbH
<120> Verfahren zur Herstellung von L-Aminosäuren
<130> 200800120 DE
<160> 21
<170> PatentIn version 3.3
<210> 1
   <211> 669
   <212> DNA
   <213> Corynebacterium glutamicum ATCC13032
<220>
   <221> CDS
   <222> (1)..(666)
   <223> amtR-Gen
<220>
   <221> misc_feature
   <222> (8)..(8)
   <223> Nukleobase G
<400> 1
   atg gca gga gca gtg gga cgc ccc cgg aga tca gct ccg cga cgg gca      48
   Met Ala Gly Ala Val Gly Arg Pro Arg Arg Ser Ala Pro Arg Arg Ala
   1               5                   10                  15
   ggc aag aat cct cgc gag gag att ctt gac gcc tct gct gag ctt ttc      96
   Gly Lys Asn Pro Arg Glu Glu Ile Leu Asp Ala Ser Ala Glu Leu Phe
               20                  25                  30
   acc cgt caa ggc ttc gca aca acc tcc acg cat caa atc gct gat gcc     144
   Thr Arg Gln Gly Phe Ala Thr Thr Ser Thr His Gln Ile Ala Asp Ala
           35                  40                  45
   gtg gga atc cgc caa gcc tcg ctg tat tat cac ttc ccg tcc aag acg     192
   Val Gly Ile Arg Gln Ala Ser Leu Tyr Tyr His Phe Pro Ser Lys Thr
       50                  55                  60
   gaa atc ttc ctc acc ctg ctg aaa tct act gtc gag ccg tcc act gtg     240
   Glu Ile Phe Leu Thr Leu Leu Lys Ser Thr Val Glu Pro Ser Thr Val
   65                  70                  75                  80
   ctc gcc gaa gac tta agc acc ctg gac gcc gga cct gag atg cgc ctc     288
   Leu Ala Glu Asp Leu Ser Thr Leu Asp Ala Gly Pro Glu Met Arg Leu
                   85                  90                  95
   tgg gca atc gtt gcc tcc gaa gtg cgt ctg ctg ctg tcc acc aag tgg     336
   Trp Ala Ile Val Ala Ser Glu Val Arg Leu Leu Leu Ser Thr Lys Trp
               100                 105                 110
   aac gtc ggt cgc ctg tac caa ctc ccc atc gtt ggt tct gaa gag ttc     384
   Asn Val Gly Arg Leu Tyr Gln Leu Pro Ile Val Gly Ser Glu Glu Phe
           115                 120                 125
   gcc gag tac cac agc cag cgc gaa gcc ctc acc aac gtc ttc cgc gac     432
   Ala Glu Tyr His Ser Gln Arg Glu Ala Leu Thr Asn Val Phe Arg Asp
       130                 135                 140
   ctc gcc acc gaa atc gtc ggt gac gac ccc cgc gca gaa ctc ccc ttc     480
   Leu Ala Thr Glu Ile Val Gly Asp Asp Pro Arg Ala Glu Leu Pro Phe
   145                 150                 155                 160
   cac atc acc atg tcg gtg atc gaa atg cgt cgc aac gac ggc aag att     528
   His Ile Thr Met Ser Val Ile Glu Met Arg Arg Asn Asp Gly Lys Ile
                   165                 170                 175
   cca agc ccg ctt tcc gca gac agc ctc ccg gag acc gca att atg ctt     576
   Pro Ser Pro Leu Ser Ala Asp Ser Leu Pro Glu Thr Ala Ile Met Leu
               180                 185                 190
   gcc gac gcc tcc ctc gcc gtc ctc ggc gcg ccg ctg ccc gcc gac cgg     624
   Ala Asp Ala Ser Leu Ala Val Leu Gly Ala Pro Leu Pro Ala Asp Arg
           195                 200                 205
   gtc gaa aaa acg ctt gaa cta atc aag cag gct gac gcg aaa taa         669
   Val Glu Lys Thr Leu Glu Leu Ile Lys Gln Ala Asp Ala Lys
       210                 215                 220
<210> 2
   <211> 222
   <212> PRT
   <213> Corynebacterium glutamicum ATCC13032
<400> 2
   Met Ala Gly Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr Arg Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Thr Val
   65                  70                  75                  80
   Leu Ala Glu Asp Leu Ser Thr Leu Asp Ala Gly Pro Glu Met Arg Leu
                   85                  90                  95
   Trp Ala Ile Val Ala Ser Glu Val Arg Leu Leu Leu Ser Thr Lys Trp
               100                 105                 110
   Asn Val Gly Arg Leu Tyr Gln Leu Pro Ile Val Gly Ser Glu Glu Phe
           115                 120                 125
   Ala Glu Tyr His Ser Gln Arg Glu Ala Leu Thr Asn Val Phe Arg Asp
       130                 135                 140
   Leu Ala Thr Glu Ile Val Gly Asp Asp Pro Arg Ala Glu Leu Pro Phe
   145                 150                 155                 160
   His Ile Thr Met Ser Val Ile Glu Met Arg Arg Asn Asp Gly Lys Ile
                   165                 170                 175
   Pro Ser Pro Leu Ser Ala Asp Ser Leu Pro Glu Thr Ala Ile Met Leu
               180                 185                 190
   Ala Asp Ala Ser Leu Ala Val Leu Gly Ala Pro Leu Pro Ala Asp Arg
           195                 200                 205
   Val Glu Lys Thr Leu Glu Leu Ile Lys Gln Ala Asp Ala Lys
       210                 215                 220
<210> 3
   <211> 2669
   <212> DNA
   <213> Corynebacterium glutamicum ATCC13032
<220>
   <221> misc_feature
   <222> (1)..(1000)
   <223> stromaufwärts der Kodierregion gelegene Sequenz
<220>
   <221> CDS
   <222> (1001)..(1666)
   <223> amtR-Gen
<220>
   <221> misc_feature
   <222> (1008)..(1008)
   <223> Nukleobase G
<220>
   <221> misc_feature
   <222> (1667)..(2669)
   <223> stromabwärts der Kodierregion gelegene Sequenz
<400> 3
   ctatgtctga ttctattggt gtcgcatgcg cagtgtagtt ttagaactac tagagaactg     60
   gaaggaatca ctcgtgcttc acccctcatt gactgagcta gcggatgctg ccccacttgc    120
   acaggatttt gccaccgtcc gcggtgtgct gaaggaatcg ctggatttgt tgggcaatgc    180
   actgaaccac ggtgaagagc ccgcggagct tgcagggtgg ctgtcacaag ttattactga    240
   tgttttgcac tcccctggct tggatgccca cgtggtgctc accggccccg tggggcgtgg     300
   agacgcactg cctacctcgc ccgtgaggtg gctggcggtc gtcgatagcc aagaagatcc     360
   gaatgaaaag atttcagcgc tgttaactga ggtgggcttc attgcggagc cgatcggtgc     420
   ggcaactcgt gaggagtggg agcagcgtgc gcgcgctggt gaggatccag aggtctattt     480
   ggatgctggc acgtgggtcg cggcgatcgc tgaagtagat gacaaagcac tgttgcagga     540
   tgcgttgtca tctaggccgc ctgcggtgga aacttatgag ggtcttcctt cgttggacat     600
   ggtggtaaac attcgtgaga acctcatgat tcccacggtg aagatcgctc gctgggcagc     660
   acacaaggct ggttctttgg cgcctacgac tgcgcagcgc ctcgtggatg cccgtggtgt     720
   gctcaccaat gatgaagtcg acgcgctgac acaggtgtgg acttccgcac tgagcttgca     780
   gtcgaaacgt tggatggatc acatccatga tcaagaaacc accgcttggg agcttcccgc     840
   gctgcaacgt gccacttttg gcgcatcggc tcggttgctt tctgaggtgt tgcggtccgt     900
   tgaagcccgt gaaatcgata ccaaatagga actctgcaca attactggct acaatctctt     960
   gagatcaata ggccaaactt taaggaagta gaattacgct atg gca gga gca gtg      1015
                                             Met Ala Gly Ala Val
                                             1               5
   gga cgc ccc cgg aga tca gct ccg cga cgg gca ggc aag aat cct cgc      1063
   Gly Arg Pro Arg Arg Ser Ala Pro Arg Arg Ala Gly Lys Asn Pro Arg
                   10                  15                  20
   gag gag att ctt gac gcc tct gct gag ctt ttc acc cgt caa ggc ttc      1111
   Glu Glu Ile Leu Asp Ala Ser Ala Glu Leu Phe Thr Arg Gln Gly Phe
               25                  30                  35
   gca aca acc tcc acg cat caa atc gct gat gcc gtg gga atc cgc caa      1159
   Ala Thr Thr Ser Thr His Gln Ile Ala Asp Ala Val Gly Ile Arg Gln
           40                  45                  50
   gcc tcg ctg tat tat cac ttc ccg tcc aag acg gaa atc ttc ctc acc      1207
   Ala Ser Leu Tyr Tyr His Phe Pro Ser Lys Thr Glu Ile Phe Leu Thr
       55                  60                  65
   ctg ctg aaa tct act gtc gag ccg tcc act gtg ctc gcc gaa gac tta      1255
   Leu Leu Lys Ser Thr Val Glu Pro Ser Thr Val Leu Ala Glu Asp Leu
   70                  75                  80                  85
   agc acc ctg gac gcc gga cct gag atg cgc ctc tgg gca atc gtt gcc      1303
   Ser Thr Leu Asp Ala Gly Pro Glu Met Arg Leu Trp Ala Ile Val Ala
                   90                  95                  100
   tcc gaa gtg cgt ctg ctg ctg tcc acc aag tgg aac gtc ggt cgc ctg      1351
   Ser Glu Val Arg Leu Leu Leu Ser Thr Lys Trp Asn Val Gly Arg Leu
               105                 110                 115
   tac caa ctc ccc atc gtt ggt tct gaa gag ttc gcc gag tac cac agc      1399
   Tyr Gln Leu Pro Ile Val Gly Ser Glu Glu Phe Ala Glu Tyr His Ser
           120                 125                 130
   cag cgc gaa gcc ctc acc aac gtc ttc cgc gac ctc gcc acc gaa atc      1447
   Gln Arg Glu Ala Leu Thr Asn Val Phe Arg Asp Leu Ala Thr Glu Ile
       135                 140                 145
   gtc ggt gac gac ccc cgc gca gaa ctc ccc ttc cac atc acc atg tcg      1495
   Val Gly Asp Asp Pro Arg Ala Glu Leu Pro Phe His Ile Thr Met Ser
   150                 155                 160                 165
   gtg atc gaa atg cgt cgc aac gac ggc aag att cca agc ccg ctt tcc      1543
   Val Ile Glu Met Arg Arg Asn Asp Gly Lys Ile Pro Ser Pro Leu Ser
                   170                 175                 180
   gca gac agc ctc ccg gag acc gca att atg ctt gcc gac gcc tcc ctc      1591
   Ala Asp Ser Leu Pro Glu Thr Ala Ile Met Leu Ala Asp Ala Ser Leu
               185                 190                 195
   gcc gtc ctc ggc gcg ccg ctg ccc gcc gac cgg gtc gaa aaa acg ctt      1639
   Ala Val Leu Gly Ala Pro Leu Pro Ala Asp Arg Val Glu Lys Thr Leu
           200                 205                 210
   gaa cta atc aag cag gct gac gcg aaa taaccatccg cgcctgcgaa            1686
   Glu Leu Ile Lys Gln Ala Asp Ala Lys
       215                 220
   atcaacggag catcaatcat ctgaccatcc agtttgaacg cacctggatg gttttctgct    1746
   tcctccacca ctttcttcgc ccactccaac tggttagcct ctggccgata ggctctccga    1806
   acaatctcga tctgcttggg gtgaatgcat gcggtgccag cgaaaccagt ccgcgcagca    1866
   tcgaccgctt ctaaatagag gccctcttca tcgtggaaat ccgcatggat ggcatcaatg    1926
   gtgaacttcc cattcgccgc cgcgtggagg tgcatcaggg cgcgtgtaag cctcatggtg    1986
   tctcggtagg agccttcatt ggactcatca cccaagaacc tagaatgagt gcctcccaag    2046
   aggtgtgtga gatcctccgc gccccagaac attccaacga ctttagggtc cgcagcaatc    2106
   tgaggaatgc tggttgcagc ctgaggggtt tcaatcatgg cgataatgtt gaggccatct    2166
   aattcctcag gcacgctgcc aagaagttta ggaaccataa caagtgtgaa atccgtggac    2226
   ttcaccatct ccacgtcagc caaaaagtgt ggatcgctcg gccctacggt tctcacaatg    2286
   gttcgtttag gatccaaccc cgattctcta atgttcctgt aggcgacctc acggtctacc    2346
   tcccctgccc catcttccaa atcaatgatg accatatcgg ccttcgatgc tgcttttgga    2406
   atgatctcag cacgtccagc tggtgcgaag agaatagctg gtccacaaat aagttcagac    2466
   atgtactcat taatacgcga aaaggcagac cttatggtct gccttcaagc gaaatcttta    2526
   aaaactttag tgagcgaagt gtcgcgcacc agtcaggtac atggtcacgc cagccttgtt    2586
   ggctgcctca atgacctcgt tgtcgcgaat ggatccacca ggctgcacaa cagcagtgat    2646
   gccagcctca gcgagaacct caa                                            2669
<210> 4
   <211> 222
   <212> PRT
   <213> Corynebacterium glutamicum ATCC13032
<400> 4
   Met Ala Gly Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr Arg Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Thr Val
   65                  70                  75                  80
   Leu Ala Glu Asp Leu Ser Thr Leu Asp Ala Gly Pro Glu Met Arg Leu
                   85                  90                  95
   Trp Ala Ile Val Ala Ser Glu Val Arg Leu Leu Leu Ser Thr Lys Trp
               100                 105                 110
   Asn Val Gly Arg Leu Tyr Gln Leu Pro Ile Val Gly Ser Glu Glu Phe
           115                 120                 125
   Ala Glu Tyr His Ser Gln Arg Glu Ala Leu Thr Asn Val Phe Arg Asp
       130                 135                 140
   Leu Ala Thr Glu Ile Val Gly Asp Asp Pro Arg Ala Glu Leu Pro Phe
   145                 150                 155                 160
   His Ile Thr Met Ser Val Ile Glu Met Arg Arg Asn Asp Gly Lys Ile
                   165                 170                 175
   Pro Ser Pro Leu Ser Ala Asp Ser Leu Pro Glu Thr Ala Ile Met Leu
               180                 185                 190
   Ala Asp Ala Ser Leu Ala Val Leu Gly Ala Pro Leu Pro Ala Asp Arg
           195                 200                 205
   Val Glu Lys Thr Leu Glu Leu Ile Lys Gln Ala Asp Ala Lys
       210                 215                 220
<210> 5
   <211> 90
   <212> DNA
   <213> Corynebacterium glutamicum
<400> 5
   gaaatcgata ccaaatagga actctgcaca attactggct acaatctctt gagatcaata     60
   ggccaaactt taaggaagta gaattacgct                                      90
<210> 6
   <211> 669
   <212> DNA
   <213> Corynebacterium glutamicum
<220>
   <221> CDS
   <222> (1)..(666)
   <223> amtR-Allel
<220>
   <221> mutation
   <222> (8)..(8)
   <223> G > A Transition
<400> 6
   atg gca gaa gca gtg gga cgc ccc cgg aga tca gct ccg cga cgg gca      48
   Met Ala Glu Ala Val Gly Arg Pro Arg Arg Ser Ala Pro Arg Arg Ala
   1               5                   10                  15
   ggc aag aat cct cgc gag gag att ctt gac gcc tct gct gag ctt ttc      96
   Gly Lys Asn Pro Arg Glu Glu Ile Leu Asp Ala Ser Ala Glu Leu Phe
               20                  25                  30
   acc cgt caa ggc ttc gca aca acc tcc acg cat caa atc gct gat gcc     144
   Thr Arg Gln Gly Phe Ala Thr Thr Ser Thr His Gln Ile Ala Asp Ala
           35                  40                  45
   gtg gga atc cgc caa gcc tcg ctg tat tat cac ttc ccg tcc aag acg     192
   Val Gly Ile Arg Gln Ala Ser Leu Tyr Tyr His Phe Pro Ser Lys Thr
       50                  55                  60
   gaa atc ttc ctc acc ctg ctg aaa tct act gtc gag ccg tcc act gtg     240
   Glu Ile Phe Leu Thr Leu Leu Lys Ser Thr Val Glu Pro Ser Thr Val
   65                  70                  75                  80
   ctc gcc gaa gac tta agc acc ctg gac gcc gga cct gag atg cgc ctc     288
   Leu Ala Glu Asp Leu Ser Thr Leu Asp Ala Gly Pro Glu Met Arg Leu
                   85                  90                  95
   tgg gca atc gtt gcc tcc gaa gtg cgt ctg ctg ctg tcc acc aag tgg     336
   Trp Ala Ile Val Ala Ser Glu Val Arg Leu Leu Leu Ser Thr Lys Trp
               100                 105                 110
   aac gtc ggt cgc ctg tac caa ctc ccc atc gtt ggt tct gaa gag ttc     384
   Asn Val Gly Arg Leu Tyr Gln Leu Pro Ile Val Gly Ser Glu Glu Phe
           115                 120                 125
   gcc gag tac cac agc cag cgc gaa gcc ctc acc aac gtc ttc cgc gac     432
   Ala Glu Tyr His Ser Gln Arg Glu Ala Leu Thr Asn Val Phe Arg Asp
       130                 135                 140
   ctc gcc acc gaa atc gtc ggt gac gac ccc cgc gca gaa ctc ccc ttc     480
   Leu Ala Thr Glu Ile Val Gly Asp Asp Pro Arg Ala Glu Leu Pro Phe
   145                 150                 155                 160
   cac atc acc atg tcg gtg atc gaa atg cgt cgc aac gac ggc aag att     528
   His Ile Thr Met Ser Val Ile Glu Met Arg Arg Asn Asp Gly Lys Ile
                   165                 170                 175
   cca agc ccg ctt tcc gca gac agc ctc ccg gag acc gca att atg ctt     576
   Pro Ser Pro Leu Ser Ala Asp Ser Leu Pro Glu Thr Ala Ile Met Leu
               180                 185                 190
   gcc gac gcc tcc ctc gcc gtc ctc ggc gcg ccg ctg ccc gcc gac cgg     624
   Ala Asp Ala Ser Leu Ala Val Leu Gly Ala Pro Leu Pro Ala Asp Arg
           195                 200                 205
   gtc gaa aaa acg ctt gaa cta atc aag cag gct gac gcg aaa taa         669
   Val Glu Lys Thr Leu Glu Leu Ile Lys Gln Ala Asp Ala Lys
       210                 215                 220
<210> 7
   <211> 222
   <212> PRT
   <213> Corynebacterium glutamicum
<400> 7
   Met Ala Glu Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr Arg Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Thr Val
   65                  70                  75                  80
   Leu Ala Glu Asp Leu Ser Thr Leu Asp Ala Gly Pro Glu Met Arg Leu
                   85                  90                  95
   Trp Ala Ile Val Ala Ser Glu Val Arg Leu Leu Leu Ser Thr Lys Trp
               100                 105                 110
   Asn Val Gly Arg Leu Tyr Gln Leu Pro Ile Val Gly Ser Glu Glu Phe
           115                 120                 125
   Ala Glu Tyr His Ser Gln Arg Glu Ala Leu Thr Asn Val Phe Arg Asp
       130                 135                 140
   Leu Ala Thr Glu Ile Val Gly Asp Asp Pro Arg Ala Glu Leu Pro Phe
   145                 150                 155                 160
   His Ile Thr Met Ser Val Ile Glu Met Arg Arg Asn Asp Gly Lys Ile
                   165                 170                 175
   Pro Ser Pro Leu Ser Ala Asp Ser Leu Pro Glu Thr Ala Ile Met Leu
               180                 185                 190
   Ala Asp Ala Ser Leu Ala Val Leu Gly Ala Pro Leu Pro Ala Asp Arg
           195                 200                 205
   Val Glu Lys Thr Leu Glu Leu Ile Lys Gln Ala Asp Ala Lys
       210                 215                 220
<210> 8
   <211> 2669
   <212> DNA
   <213> Corynebacterium glutamicum
<220>
   <221> misc_feature
   <222> (1)..(1000)
<220>
   <221> CDS
   <222> (1001)..(1666)
   <223> amtR-Allel
<220>
   <221> mutation
   <222> (1008)..(1008)
   <223> G > A Transition
<220>
   <221> misc_feature
   <222> (1667)..(2669)
<400> 8
   ctatgtctga ttctattggt gtcgcatgcg cagtgtagtt ttagaactac tagagaactg      60
   gaaggaatca ctcgtgcttc acccctcatt gactgagcta gcggatgctg ccccacttgc     120
   acaggatttt gccaccgtcc gcggtgtgct gaaggaatcg ctggatttgt tgggcaatgc     180
   actgaaccac ggtgaagagc ccgcggagct tgcagggtgg ctgtcacaag ttattactga     240
   tgttttgcac tcccctggct tggatgccca cgtggtgctc accggccccg tggggcgtgg     300
   agacgcactg cctacctcgc ccgtgaggtg gctggcggtc gtcgatagcc aagaagatcc     360
   gaatgaaaag atttcagcgc tgttaactga ggtgggcttc attgcggagc cgatcggtgc     420
   ggcaactcgt gaggagtggg agcagcgtgc gcgcgctggt gaggatccag aggtctattt     480
   ggatgctggc acgtgggtcg cggcgatcgc tgaagtagat gacaaagcac tgttgcagga     540
   tgcgttgtca tctaggccgc ctgcggtgga aacttatgag ggtcttcctt cgttggacat     600
   ggtggtaaac attcgtgaga acctcatgat tcccacggtg aagatcgctc gctgggcagc     660
   acacaaggct ggttctttgg cgcctacgac tgcgcagcgc ctcgtggatg cccgtggtgt     720
   gctcaccaat gatgaagtcg acgcgctgac acaggtgtgg acttccgcac tgagcttgca     780
   gtcgaaacgt tggatggatc acatccatga tcaagaaacc accgcttggg agcttcccgc     840
   gctgcaacgt gccacttttg gcgcatcggc tcggttgctt tctgaggtgt tgcggtccgt     900
   tgaagcccgt gaaatcgata ccaaatagga actctgcaca attactggct acaatctctt     960
   gagatcaata ggccaaactt taaggaagta gaattacgct atg gca gaa gca gtg      1015
                                              Met Ala Glu Ala Val
                                              1               5
   gga cgc ccc cgg aga tca gct ccg cga cgg gca ggc aag aat cct cgc      1063
   Gly Arg Pro Arg Arg Ser Ala Pro Arg Arg Ala Gly Lys Asn Pro Arg
                   10                  15                  20
   gag gag att ctt gac gcc tct gct gag ctt ttc acc cgt caa ggc ttc      1111
   Glu Glu Ile Leu Asp Ala Ser Ala Glu Leu Phe Thr Arg Gln Gly Phe
               25                  30                  35
   gca aca acc tcc acg cat caa atc gct gat gcc gtg gga atc cgc caa      1159
   Ala Thr Thr Ser Thr His Gln Ile Ala Asp Ala Val Gly Ile Arg Gln
           40                  45                  50
   gcc tcg ctg tat tat cac ttc ccg tcc aag acg gaa atc ttc ctc acc      1207
   Ala Ser Leu Tyr Tyr His Phe Pro Ser Lys Thr Glu Ile Phe Leu Thr
       55                  60                  65
   ctg ctg aaa tct act gtc gag ccg tcc act gtg ctc gcc gaa gac tta      1255
   Leu Leu Lys Ser Thr Val Glu Pro Ser Thr Val Leu Ala Glu Asp Leu
   70                  75                  80                  85
   agc acc ctg gac gcc gga cct gag atg cgc ctc tgg gca atc gtt gcc      1303
   Ser Thr Leu Asp Ala Gly Pro Glu Met Arg Leu Trp Ala Ile Val Ala
                   90                  95                  100
   tcc gaa gtg cgt ctg ctg ctg tcc acc aag tgg aac gtc ggt cgc ctg      1351
   Ser Glu Val Arg Leu Leu Leu Ser Thr Lys Trp Asn Val Gly Arg Leu
               105                 110                 115
   tac caa ctc ccc atc gtt ggt tct gaa gag ttc gcc gag tac cac agc      1399
   Tyr Gln Leu Pro Ile Val Gly Ser Glu Glu Phe Ala Glu Tyr His Ser
           120                 125                 130
   cag cgc gaa gcc ctc acc aac gtc ttc cgc gac ctc gcc acc gaa atc      1447
   Gln Arg Glu Ala Leu Thr Asn Val Phe Arg Asp Leu Ala Thr Glu Ile
       135                 140                 145
   gtc ggt gac gac ccc cgc gca gaa ctc ccc ttc cac atc acc atg tcg      1495
   Val Gly Asp Asp Pro Arg Ala Glu Leu Pro Phe His Ile Thr Met Ser
   150                 155                 160                 165
   gtg atc gaa atg cgt cgc aac gac ggc aag att cca agc ccg ctt tcc      1543
   Val Ile Glu Met Arg Arg Asn Asp Gly Lys Ile Pro Ser Pro Leu Ser
                   170                 175                 180
   gca gac agc ctc ccg gag acc gca att atg ctt gcc gac gcc tcc ctc      1591
   Ala Asp Ser Leu Pro Glu Thr Ala Ile Met Leu Ala Asp Ala Ser Leu
               185                 190                 195
   gcc gtc ctc ggc gcg ccg ctg ccc gcc gac cgg gtc gaa aaa acg ctt      1639
   Ala Val Leu Gly Ala Pro Leu Pro Ala Asp Arg Val Glu Lys Thr Leu
           200                 205                 210
   gaa cta atc aag cag gct gac gcg aaa taaccatccg cgcctgcgaa            1686
   Glu Leu Ile Lys Gln Ala Asp Ala Lys
       215                 220
   atcaacggag catcaatcat ctgaccatcc agtttgaacg cacctggatg gttttctgct   1746
   tcctccacca ctttcttcgc ccactccaac tggttagcct ctggccgata ggctctccga   1806
   acaatctcga tctgcttggg gtgaatgcat gcggtgccag cgaaaccagt ccgcgcagca   1866
   tcgaccgctt ctaaatagag gccctcttca tcgtggaaat ccgcatggat ggcatcaatg   1926
   gtgaacttcc cattcgccgc cgcgtggagg tgcatcaggg cgcgtgtaag cctcatggtg   1986
   tctcggtagg agccttcatt ggactcatca cccaagaacc tagaatgagt gcctcccaag   2046
   aggtgtgtga gatcctccgc gccccagaac attccaacga ctttagggtc cgcagcaatc   2106
   tgaggaatgc tggttgcagc ctgaggggtt tcaatcatgg cgataatgtt gaggccatct   2166
   aattcctcag gcacgctgcc aagaagttta ggaaccataa caagtgtgaa atccgtggac   2226
   ttcaccatct ccacgtcagc caaaaagtgt ggatcgctcg gccctacggt tctcacaatg   2286
   gttcgtttag gatccaaccc cgattctcta atgttcctgt aggcgacctc acggtctacc   2346
   tcccctgccc catcttccaa atcaatgatg accatatcgg ccttcgatgc tgcttttgga   2406
   atgatctcag cacgtccagc tggtgcgaag agaatagctg gtccacaaat aagttcagac   2466
   atgtactcat taatacgcga aaaggcagac cttatggtct gccttcaagc gaaatcttta   2526
   aaaactttag tgagcgaagt gtcgcgcacc agtcaggtac atggtcacgc cagccttgtt   2586
   ggctgcctca atgacctcgt tgtcgcgaat ggatccacca ggctgcacaa cagcagtgat   2646
   gccagcctca gcgagaacct caa                                            2669
<210> 9
   <211> 222
   <212> PRT
   <213> Corynebacterium glutamicum
<400> 9
   Met Ala Glu Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr Arg Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Thr Val
   65                  70                  75                  80
   Leu Ala Glu Asp Leu Ser Thr Leu Asp Ala Gly Pro Glu Met Arg Leu
                   85                  90                  95
   Trp Ala Ile Val Ala Ser Glu Val Arg Leu Leu Leu Ser Thr Lys Trp
               100                 105                 110
   Asn Val Gly Arg Leu Tyr Gln Leu Pro Ile Val Gly Ser Glu Glu Phe
           115                 120                 125
   Ala Glu Tyr His Ser Gln Arg Glu Ala Leu Thr Asn Val Phe Arg Asp
       130                 135                 140
   Leu Ala Thr Glu Ile Val Gly Asp Asp Pro Arg Ala Glu Leu Pro Phe
   145                 150                 155                 160
   His Ile Thr Met Ser Val Ile Glu Met Arg Arg Asn Asp Gly Lys Ile
                   165                 170                 175
   Pro Ser Pro Leu Ser Ala Asp Ser Leu Pro Glu Thr Ala Ile Met Leu
               180                 185                 190
   Ala Asp Ala Ser Leu Ala Val Leu Gly Ala Pro Leu Pro Ala Asp Arg
           195                 200                 205
   Val Glu Lys Thr Leu Glu Leu Ile Lys Gln Ala Asp Ala Lys
       210                 215                 220
<210> 10
   <211> 672
   <212> DNA
   <213> Corynebacterium efficiens
<220>
   <221> CDS
   <222> (1)..(669)
   <223> amtR-Gen
<400> 10
   atg gca ggt gca gtc gga cgc ccc cgg agg tca gct ccg cgt cgg gcg      48
   Met Ala Gly Ala Val Gly Arg Pro Arg Arg Ser Ala Pro Arg Arg Ala
   1               5                   10                  15
   ggc aag aat ccc cgc gag gag att ctc gat gcc tca gct gag ctg ttc      96
   Gly Lys Asn Pro Arg Glu Glu Ile Leu Asp Ala Ser Ala Glu Leu Phe
               20                  25                  30
   acc cgc cag ggt ttc gcc acc acc tcc acg cac cag atc gcc gat gcc     144
   Thr Arg Gln Gly Phe Ala Thr Thr Ser Thr His Gln Ile Ala Asp Ala
           35                  40                  45
   gtg ggt atc cgg cag gcc tcc ctg tac tac cac ttc ccg tcc aag acg     192
   Val Gly Ile Arg Gln Ala Ser Leu Tyr Tyr His Phe Pro Ser Lys Thr
       50                  55                  60
   gag atc ttc ctc acc ctg ctg aaa tcc acc gtc gag cca tcc atg gtg     240
   Glu Ile Phe Leu Thr Leu Leu Lys Ser Thr Val Glu Pro Ser Met Val
   65                  70                  75                  80
   ttg gcc ggc gac ctg gcc aat ctc gag gcc tcc ccg gag ctg cgc ctg     288
   Leu Ala Gly Asp Leu Ala Asn Leu Glu Ala Ser Pro Glu Leu Arg Leu
                   85                  90                  95
   tgg gca ctg gtg gcg gcc gag gtg cgt cta ctg ctg tcg acg aag tgg     336
   Trp Ala Leu Val Ala Ala Glu Val Arg Leu Leu Leu Ser Thr Lys Trp
               100                 105                 110
   aat gtc ggt cgt ctc tat caa ctg ccg atc gtg gcc tcc gag gag ttc     384
   Asn Val Gly Arg Leu Tyr Gln Leu Pro Ile Val Ala Ser Glu Glu Phe
           115                 120                 125
   gag gag tac cac acg cag cgt gcc acc ctg acg gat acc ttc cgc agc     432
   Glu Glu Tyr His Thr Gln Arg Ala Thr Leu Thr Asp Thr Phe Arg Ser
       130                 135                 140
   ctg gcc acg gag atc gtc ggt gag gat gac ccc cgt gcg gaa ctc ccg     480
   Leu Ala Thr Glu Ile Val Gly Glu Asp Asp Pro Arg Ala Glu Leu Pro
   145                 150                 155                 160
   ttc cac atc acg atg tcc gcc atc gag atg cgc cgc aat gac ggc aag     528
   Phe His Ile Thr Met Ser Ala Ile Glu Met Arg Arg Asn Asp Gly Lys
                   165                 170                 175
   gtt ccc agc ccc ctg tcg gag gac agc ctc ccg gac acc gcc gtc atg     576
   Val Pro Ser Pro Leu Ser Glu Asp Ser Leu Pro Asp Thr Ala Val Met
               180                 185                 190
   ctt gcc gac gcc gcc ctc gcc gtc ctg ggg gcc gac ctg ccc ggg gac     624
   Leu Ala Asp Ala Ala Leu Ala Val Leu Gly Ala Asp Leu Pro Gly Asp
           195                 200                 205
   cgg gtg gag cgc acc ctg gaa ctg ctc agg cag gct gac gcg aaa taa      672
   Arg Val Glu Arg Thr Leu Glu Leu Leu Arg Gln Ala Asp Ala Lys
       210                 215                 220
<210> 11
   <211> 223
   <212> PRT
   <213> Corynebacterium efficiens
<400> 11
   Met Ala Gly Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr Arg Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Met Val
   65                  70                  75                  80
   Leu Ala Gly Asp Leu Ala Asn Leu Glu Ala Ser Pro Glu Leu Arg Leu
                   85                  90                  95
   Trp Ala Leu Val Ala Ala Glu Val Arg Leu Leu Leu Ser Thr Lys Trp
               100                 105                 110
   Asn Val Gly Arg Leu Tyr Gln Leu Pro Ile Val Ala Ser Glu Glu Phe
           115                 120                 125
   Glu Glu Tyr His Thr Gln Arg Ala Thr Leu Thr Asp Thr Phe Arg Ser
       130                 135                 140
   Leu Ala Thr Glu Ile Val Gly Glu Asp Asp Pro Arg Ala Glu Leu Pro
   145                 150                 155                 160
   Phe His Ile Thr Met Ser Ala Ile Glu Met Arg Arg Asn Asp Gly Lys
                   165                 170                 175
   Val Pro Ser Pro Leu Ser Glu Asp Ser Leu Pro Asp Thr Ala Val Met
               180                 185                 190
   Leu Ala Asp Ala Ala Leu Ala Val Leu Gly Ala Asp Leu Pro Gly Asp
               195                 200                 205
   Arg Val Glu Arg Thr Leu Glu Leu Leu Arg Gln Ala Asp Ala Lys
       210                 215                 220
<210> 12
   <211> 1266
   <212> DNA
   <213> Corynebacterium glutamicum
<220>
   <221> CDS
   <222> (1)..(1263)
   <223> lysC-Wildtyp-Gen
<400> 12
   gtg gcc ctg gtc gta cag aaa tat ggc ggt tcc tcg ctt gag agt gcg       48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct       96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat      144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt      192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc      240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg      288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc      336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc      384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc      432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg      480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt      528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc taa                                                   1266
   Ala Gly Thr Gly Arg
               420
<210> 13
   <211> 421
   <212> PRT
   <213> Corynebacterium glutamicum
<400> 13
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<211> 28
   <212> DNA
   <213> Corynebacterium glutamicum
<220>
   <221> misc_feature
   <223> AmtR-Bindestelle stromaufwärts des amtA-Gens
<400> 14
   tttttaccta tcgttctata gatttctg 28
<210> 15
   <211> 28
   <212> DNA
   <213> Corynebacterium glutamicum
<220>
   <221> misc_feature
   <223> AmtR-Bindestelle stromaufwärts des amtA-Gens
<400> 15
   gtattttcta ttccgctata gataaacc 28
<210> 16
   <211> 28
   <212> DNA
   <213> Corynebacterium glutamicum
<220>
   <221> misc_feature
   <223> AmtR-Bindestelle stromaufwärts des amtB-Gens
<400> 16
   atattttcta tagtttaaca ggtaattt 28
<210> 17
   <211> 28
   <212> DNA
   <213> Corynebacterium glutamicum
<220>
   <221> misc_feature
   <223> AmtR-Bindestelle stromaufwärts des amtB-Gens
<400> 17
   gctctaacta tagacctaca gaaactaa 28
<210> 18
   <211> 28
   <212> DNA
   <213> Corynebacterium glutamicum
<220>
   <221> misc_feature
   <223> AmtR-Bindestelle stromaufwärts des gltB-Gens
<400> 18
   cgttttccta taggttgatc gaaagtaa 28
<210> 19
   <211> 28
   <212> DNA
   <213> Corynebacterium glutamicum
<220>
   <221> misc_feature
   <223> AmtR-Bindestelle stromaufwärts des gltB-Gens
<400> 19
   ttattatcga acgattgata gaaacagg 28
<210> 20
   <211> 669
   <212> DNA
   <213> Corynebacterium glutamicum ATCC14067
<220>
   <221> CDS
   <222> (1)..(666)
<400> 20
   atg gca gga gca gtg gga cgc ccc cgg aga tca gct ccg cga cgg gca       48
   Met Ala Gly Ala Val Gly Arg Pro Arg Arg Ser Ala Pro Arg Arg Ala
   1               5                   10                  15
   ggc aag aat cct cgc gag gag att ctt gac gcc tct gct gag ctt ttc       96
   Gly Lys Asn Pro Arg Glu Glu Ile Leu Asp Ala Ser Ala Glu Leu Phe
               20                  25                  30
   acc cat caa ggc ttc gca aca acc tcc acg cat caa atc gct gat gcc      144
   Thr His Gln Gly Phe Ala Thr Thr Ser Thr His Gln Ile Ala Asp Ala
           35                  40                  45
   gtg gga atc cgc caa gcc tcg ctg tat tat cac ttc ccg tct aag acg      192
   Val Gly Ile Arg Gln Ala Ser Leu Tyr Tyr His Phe Pro Ser Lys Thr
       50                  55                  60
   gaa atc ttc ctc acc ctc ctg aaa tct acc gtc gag ccg tcc act gtg      240
   Glu Ile Phe Leu Thr Leu Leu Lys Ser Thr Val Glu Pro Ser Thr Val
   65                  70                  75                  80
   ctc gcc gaa gac tta agc atc ctg gat gca gga cct gag atg cgc ctc      288
   Leu Ala Glu Asp Leu Ser Ile Leu Asp Ala Gly Pro Glu Met Arg Leu
                   85                  90                  95
   tgg gca atc gtt gcc tcc gaa gtg cgt ctg ctg ctg tcc acc aag tgg      336
   Trp Ala Ile Val Ala Ser Glu Val Arg Leu Leu Leu Ser Thr Lys Trp
               100                 105                 110
   aac gtc ggt cgc ctg tac caa ctc ccc atc gtt ggt tct gaa gag ttc      384
   Asn Val Gly Arg Leu Tyr Gln Leu Pro Ile Val Gly Ser Glu Glu Phe
           115                 120                 125
   gcc gag tac cac agc cag cgc gaa gcc ctc acc aac gtc ttc cgc gac      432
   Ala Glu Tyr His Ser Gln Arg Glu Ala Leu Thr Asn Val Phe Arg Asp
       130                 135                 140
   ctc gcc acc gaa atc gtc ggt gac gac ccc cgc gca gaa ctc ccc ttc      480
   Leu Ala Thr Glu Ile Val Gly Asp Asp Pro Arg Ala Glu Leu Pro Phe
   145                 150                 155                 160
   cac atc acc atg tcg gtg atc gaa atg cgt cgc aac gac ggc aag att      528
   His Ile Thr Met Ser Val Ile Glu Met Arg Arg Asn Asp Gly Lys Ile
                   165                 170                 175
   cca agc ccg ctt tcc gca gac agc ctc ccg gag acc gca att atg ctt      576
   Pro Ser Pro Leu Ser Ala Asp Ser Leu Pro Glu Thr Ala Ile Met Leu
               180                 185                 190
   gcc gac gcc tcc ctc gcc gtc ctc ggc gcg tcg ctg ccc gcc gac cgg      624
   Ala Asp Ala Ser Leu Ala Val Leu Gly Ala Ser Leu Pro Ala Asp Arg
           195                 200                 205
   gtc gaa aaa acg ctt gaa cta atc aag cag gct gac gcg aaa taa          669
   Val Glu Lys Thr Leu Glu Leu Ile Lys Gln Ala Asp Ala Lys
       210                 215                 220
<210> 21
   <211> 222
   <212> PRT
   <213> Corynebacterium glutamicum ATCC14067
<400> 21
   Met Ala Gly Ala Val Gly Arg Pro Arg Arg Ser Ala Pro Arg Arg Ala
   1               5                   10                  15
   Gly Lys Asn Pro Arg Glu Glu Ile Leu Asp Ala Ser Ala Glu Leu Phe
               20                  25                  30
   Thr His Gln Gly Phe Ala Thr Thr Ser Thr His Gln Ile Ala Asp Ala
           35                  40                  45
   Val Gly Ile Arg Gln Ala Ser Leu Tyr Tyr His Phe Pro Ser Lys Thr
       50                  55                  60
   Glu Ile Phe Leu Thr Leu Leu Lys Ser Thr Val Glu Pro Ser Thr Val
   65                  70                  75                  80
   Leu Ala Glu Asp Leu Ser Ile Leu Asp Ala Gly Pro Glu Met Arg Leu
                   85                  90                  95
   Trp Ala Ile Val Ala Ser Glu Val Arg Leu Leu Leu Ser Thr Lys Trp
               100                 105                 110
   Asn Val Gly Arg Leu Tyr Gln Leu Pro Ile Val Gly Ser Glu Glu Phe
           115                 120                 125
   Ala Glu Tyr His Ser Gln Arg Glu Ala Leu Thr Asn Val Phe Arg Asp
       130                 135                 140
   Leu Ala Thr Glu Ile Val Gly Asp Asp Pro Arg Ala Glu Leu Pro Phe
   145                 150                 155                 160
   His Ile Thr Met Ser Val Ile Glu Met Arg Arg Asn Asp Gly Lys Ile
                   165                 170                 175
   Pro Ser Pro Leu Ser Ala Asp Ser Leu Pro Glu Thr Ala Ile Met Leu
               180                 185                 190
   Ala Asp Ala Ser Leu Ala Val Leu Gly Ala Ser Leu Pro Ala Asp Arg
           195                 200                 205
   Val Glu Lys Thr Leu Glu Leu Ile Lys Gln Ala Asp Ala Lys
       210                 215                 220